# EUROPEAN PATENT SPECIFICATION

(11) **EP 4 423 293 B1**
(45) Date of publication and mention of the grant of the patent: **11.11.2026**
(21) Application number: 22800859.5
(22) Date of filing: 25.10.2022
(51) Int. Cl.: C12Q 1/6806

(54) **METHOD**
VERFAHREN
PROCÉDÉ

(30) Priority: 25.10.2021 GB 202115325
(43) Date of publication of application: 04.09.2024
(73) Proprietor: Countagen AB, 17165 Solna (SE)
(72) Inventor: NEUMANN, Felix, 12050 Stockholm (SE); ASTOBIZA, Leiore Ajuria, 11346 Stockholm (SE); BERGMAN, Joost, 11768 Stockholm (SE)
(74) Representative: Potter Clarkson
(86) International application number: PCT/SE2022/050974
(87) International publication number: WO 2023/075663

(56) References cited:
- WO-A2-2007/092941
- WO-A2-2008/109207
- WO-A2-2009/037659
- CN-B- 111 575 351
- MINERO GABRIEL ANTONIO ET AL: "Integration of microbead DNA handling with optomagnetic detection in rolling circle amplification assays", MICROCHIMICA ACTA, SPRINGER VIENNA, VIENNA, vol. 186, no. 8, 11 July 2019 (2019-07-11), pages 1 - 10, XP036868525, ISSN: 0026-3672, [retrieved on 20190711], DOI: 10.1007/S00604-019-3636-X
- GARBARINO FRANCESCA ET AL: "Integration of rolling circle amplification and optomagnetic detection on a polymer chip", BIOSENSORS AND BIOELECTRONICS, ELSEVIER SCIENCE LTD, UK, AMSTERDAM , NL, vol. 142, 3 July 2019 (2019-07-03), XP085794044, ISSN: 0956-5663, [retrieved on 20190703], DOI: 10.1016/J.BIOS.2019.111485
- MEZGER ANJA ET AL: "Scalable DNA-Based Magnetic Nanoparticle Agglutination Assay for Bacterial Detection in Patient Samples", ACS NANO, vol. 9, no. 7, 28 July 2015 (2015-07-28), US, pages 7374 - 7382, XP055898114, ISSN: 1936-0851, Retrieved from the Internet <URL:https://pubs.acs.org/doi/pdf/10.1021/acsnano.5b02379> DOI: 10.1021/acsnano.5b02379
- MALTE KÜHNEMUND ET AL: "Circle-to-circle amplification on a digital microfluidic chip for amplified single molecule detection", LAB ON A CHIP, vol. 14, no. 16, 1 January 2014 (2014-01-01), UK, pages 2983 - 2992, XP055403238, ISSN: 1473-0197, DOI: 10.1039/C4LC00348A
- KÜHNEMUND MALTE ET AL: "Sensitive and inexpensive digital DNA analysis by microfluidic enrichment of rolling circle amplified single-molecules", NUCLEIC ACIDS RESEARCH, vol. 45, 1 January 2017 (2017-01-01), GB, pages gkw1324, XP093014972, ISSN: 0305-1048, Retrieved from the Internet <URL:https://academic.oup.com/nar/article-pdf/45/8/e59/25366365/gkw1324.pdf> DOI: 10.1093/nar/gkw1324

## Description

### Technical Field

The present invention relates to a sample analysis method, and in particular to such a sample analysis method for measuring, analyzing and quantifying polynucleotides and/or oligonucleotides, such as rolling circle amplification (RCA) products (RCPs).

### Prior Art and Background

The precise quantification of biomolecules, in particular of nucleic acids, is of paramount importance for biomedical research, genetic engineering and drug development. Single molecule solutions have proven to be superior to bulk measurements as they allow to detect subtle differences in amounts, e.g., digital polymerase chain reaction (PCR) has many advantages over classical PCR.

RCA is a single molecule amplification technique that can be used to detect individual copies of molecules. RCA is inherently digital, meaning it does not require compartmentalization into droplets or wells as digital PCR does, to be able to distinguish single molecule copies in a complex solution. RCPs are most often detected by an optical sensor when being labeled with fluorophores. However, other optical and non-optical readout modes have been explored as well. A major challenge for quantifying RCPs from a liquid sample containing RCPs is to match the final reaction volume with the focal volume of the optical device. This creates a mismatch, while the absolute numbers of RCPs in a sample may be sufficiently high to detect, the concentration of RCPs in the sample may be low, which might require that the entire sample volume has to be analyzed in order to detect all, or a substantial fraction of all RPCs in the liquid sample to reach statistical significance.

RCPs in a liquid sample can be applied and spread onto a 2-dimensional (2D) surface, such as a glass slide, and the total number of RCPs can then be determined by imaging the entire glass slide. Such a procedure, however, requires a sophisticated automated microscope with scanning stage that acquires images of several adjacent fields of view of the microscope optical objective with high precision so that the entire area can be captured.

Capturing nucleic acids on beads has been shown to be useful for a variety of applications.

For example, Sato et al. Microbead-based rolling circle amplification in a microchip for sensitive DNA detection. Lab Chip (2010); 10:1262-1266 describes the use of microbeads for the amplification of RCPs on beads and the subsequent digital quantification. However, this system required loading with reactions, and the bead-bound products are unable to be easily concentrated into a small surface area. In another example, Soares et al. Silica bead-based microfluidic device with integrated photodiodes for the rapid capture and detection of rolling circle amplification products in the femtomolar range, Biosens. Bioelectron. (2019), 1;128:68-75 describes the trapping of RCPs on silica microbeads for a fluorescence intensity-based readout. However, this system required continuous flow, uses large beads of several tens of micro-meter size and does not allow for digital quantification of RCPs. Yet another example of Donolato et al. Quantification of rolling circle amplified DNA using magnetic nanobeads and a Blu-ray optical pick-up unit. Biosens. Bioelectron. (2014); 67:649-655 discloses the use of nanobeads for the capture of RCPs and subsequent opto-magnetic quantification. However, RCPs are bound to multiple magnetic beads to increase the magnetic momentum and a digital quantification of single RCPs is not possible. In summary, none of these methods have described the possibility to concentrate bead-bound RCPs in a small area in order to digitally quantify the nucleic acids in a single field of view. Furthermore, the increased fluorescence intensity observed of bead-bound RCPs has not been described.

WO 2007/092941 relates to methods and systems for the use of Surface Enhanced Raman Scattering nanotags (SERS nanotags). Minero et al., 2019, Mikrochimica Acta, 186(8):528 relates to methods for the integration of microbead DNA handling with optomagnetic detection in rolling circle amplification assays.

Presented herein is a new method using magnetic beads to capture (or generate on them) polynucleotides and/or oligonucleotides in a liquid sample, and concentrating them into, or towards, a small surface area using a magnetic source. This method allows to maintain the number of polynucleotides/oligonucleotides originally in the sample volume and effectively increases the local concentration of polynucleotides/oligonucleotides into a single field of view of an optical sensing device, such as a microscope objective. The sample analysis method facilitates analysis of samples containing RCPs with simple optical readout, while still achieving a high detection sensitivity.

Also presented herein are sample analysis devices for use in the method.

### Disclosure of the Invention

The present invention is defined by the accompanying claims.

According to a first aspect of the invention there is provided a method of analyzing a sample comprising of a plurality of polynucleotides and/or oligonucleotides of interest, wherein the method comprises:
(i) providing a sample solution comprising a plurality of polynucleotides and/or oligonucleotides of interest;
(ii) attaching the polynucleotides/oligonucleotides to magnetic beads to provide bead-bound polynucleotides/oligonucleotides, thereby providing a further sample solution;
(iii) applying the further sample solution to a first surface of a sample support element; and
(iv) providing a magnetic source so as to draw (e.g., attract) the bead-bound polynucleotides/oligonucleotides to a position on the first surface of the sample support element,
   wherein the magnetic source comprises an array of magnets or electromagnets, or a combination thereof, that are spatially configured around the sample support element so as to produce focused magnetic fields that draw (e.g., attract) the bead-bound polynucleotides/oligonucleotides to a position on the first surface of the sample support element;
wherein following step (iv) the bead-bound polynucleotides/oligonucleotides are visualized and/or quantified using a microscope; and
wherein the magnetic beads have an average size of from about 30 nm to about 200 nm, and which method is referred to hereinafter as "the method of the invention".

It is an object of the present disclosure to overcome or at least mitigate one or more of the problems discussed above, and to provide advantages and aspects not provided by hitherto known techniques.

A particular objective of the method of the invention is to enable the concentration and focus of polynucleotides/oligonucleotides from the further sample solution onto/into a small defined area. This and other objectives are met by the invention as disclosed herein.

To explain further, in step (iv) where it is stated that the magnetic source draws (e.g. attracts) the bead-bound polynucleotides/oligonucleotides to a position on the first surface of the sample support element, this means that prior to providing the magnetic source the bead-bound polynucleotides/oligonucleotides are distributed within the further sample solution as it is applied on the first surface of the sample support element. Following the provision of the magnetic source, the magnetic beads are drawn (e.g., attracted) towards a pre-determined position of the first surface of the sample support element. For the avoidance of doubt, it is not necessary for the magnetic beads to be in contact with the first surface of the sample support element for the invention to be put into practice, so long as the magnetic beads are drawn (e.g. attracted) towards the area to allow analysis and/or visualization.

By the term "drawn" we include that the bead-bound polynucleotides/oligonucleotides are "attracted" to a position on the first surface of the sample by the magnetic source, or that the bead-bound polynucleotides/oligonucleotides are "repelled" to a position on the first surface of the sample by the magnetic source. Indeed, the bead-bound polynucleotides/oligonucleotides may be drawn to the position by a combination of attractive and repellant forces provided by an arrangement of multiple magnetic sources, such that the combination of forces provides a focal point towards which the bead-bound polynucleotides/oligonucleotides are drawn. The term "draw" as used herein may be replaced with either "attract" or "repel".

That is to say, in step (iv) the magnetic source may be provided so as to attract the bead-bound polynucleotides/oligonucleotides to a position on the first surface of the sample support element.

In step (iv), the magnetic source may be provided at a second surface of the sample support element opposite to the first surface. By this we refer to a magnetic source, for example a magnet, being in contact with the second surface of the sample support element.

Alternatively, the magnetic source may be provided in the vicinity of the sample support element so as to draw (e.g. attract) the bead-bound polynucleotides/oligonucleotides to a position on the first surface of the sample support element. By this we mean that a magnetic source, or indeed multiple magnetic sources, is/are provided close enough to the sample support element so that their magnetic fields are focused so as to draw (e.g., attract) the bead-bound polynucleotides/oligonucleotides to a position on the first surface of the sample support element. This means that the magnetic source need not necessarily be in contact with the sample support element to put the invention into practice. For example, the magnetic source may be an array of magnets or electromagnets, or a combination thereof, that are spatially configured around the sample support element so as to produce focused magnetic fields that draw (e.g., attract) the bead-bound polynucleotides/oligonucleotides to a position on the first surface of the sample support element.

Furthermore, the magnetic source may be positioned in the vicinity of a second surface of the sample support element opposite to the first surface or indeed may be positioned in the vicinity of the first surface of the sample support element.

The term "polynucleotides", as used herein, refers to a biopolymer composed of nucleotide monomers in a chain, for example DNA and/or cDNA and/or RNA. Typically, polynucleotides comprise at least 14 nucleotides in a chain.

The term "oligonucleotides", as used herein, refers to any short single strands of synthetic DNA or RNA. Typically, oligonucleotides comprise about three to twenty nucleotides in a chain.

As used herein, the term "plurality" refers to at least two of the features of interest. For example, a plurality of polynucleotides/oligonucleotides in the sample solution means that the sample solution contains at least two polynucleotides/oligonucleotides. Furthermore, the plurality of polynucleotides/oligonucleotides may be identical, or indeed the sample solution may comprise a plurality of different polynucleotides/oligonucleotides for analysis.

The skilled person will understand that the phrase "the polynucleotide and/or oligonucleotides of interest" as used herein refers to the polynucleotides and/or oligonucleotides which are to be amplified and/or analysed. The skilled person will understand that such polynucleotides and/or oligonucleotides may refer to synthetic and/or naturally occurring polynucleotides and/or oligonucleotides.

For the avoidance of doubt, when we refer to polynucleotides/oligonucleotides herein without the term "plurality" we are referring to the plurality of polynucleotides and/or oligonucleotides.

Magnetic beads may have an average size of from about 10 nm to about 5 µm, for example from about 10 nm to about 2 µm, such as about 500 nm to about 2 µm, and may have an average diameter from about 10 nm to about 5 µm, for example from about 10 nm to about 2 µm, such as about 500 nm to about 2 µm, or about 10 nm to about 1 µm, such as about 10 nm to about 500 nm. In the present invention, the magnetic beads are as defined in the accompanying claims and have an average size of from about 30 nm to about 200 nm, or about 50 nm to about 200 nm.

The coefficient of variation (CV), also commonly referred to as the relative standard of deviation (RSD), of the size of the magnetic beads may be less than about 10%, such as less than about 5 %.

The skilled person is aware of suitable methods for determining the size of magnetic beads in the nm to µm range and such methods include, but are in no way limited to, dynamic light scattering (DLS), transmission electron microscopy (TEM) scattering electron microscopy (SEM), atomic force microscopy (AFM) and laser diffraction analysis.

As used herein, the term "magnetic beads" refers to beads which are magnetic and/or possess magnetic properties.

The magnetic beads may be ferrimagnetic or superparamagnetic. It is preferred that the magnetic beads are superparamagnetic.

The magnetic beads may comprise iron, nickel, cobalt, or combinations thereof. Preferably, the magnetic beads comprise iron oxide, such as magnetite (Fe₃O₄).

Examples of magnetic beads that may be used include Dynabeads (e.g. Dynabeads^{™} MyOne^{™} Streptavidin T1 (Thermo Fisher Scientific), Dynabeads^{™}MyOne^{™} Streptavidin C1 (Thermo Fisher Scientific), Dynabeads^{™}M-270 Streptavidin (Thermo Fisher Scientific), Dynabeads^{™}M-280 Streptavidin (Thermo Fisher Scientific), Dynabeads^{™}MyOne^{™} Silane (Thermo Fisher Scientific)), MACS^{®} MicroBeads and MACSxpress^{®} Beads (Miltenyi Biotec), Turbobeads (Turbobeads Llc), Sera-Mag^{™} beads (Cytiva), Ni-NTA Magnetic Agarose Beads (QIAGEN), SuperMag Streptavidin magnetic beads (Ocean NanoTech) and MagSi (AMSBIO).

It is to be understood by the skilled person that "attaching" the polynucleotides and/or oligonucleotides to magnetic beads may include the binding of such polynucleotides and/or oligonucleotides using standard methods in the field, such as *via* adsorption and/or conjugation, or a combination thereof. It is preferred that the attachment is carried out *via* conjugation.

In the case of attachment of the polynucleotides and/or oligonucleotides to magnetic beads *via* conjugation, such conjugation may be either directly or indirectly (e.g. via a complementary capture oligonucleotide) to the polynucleotide and/or oligopeptide of interest.

The magnetic beads may comprise surface coatings and/or modifications configured for enabling the attachment of polynucleotides and/or oligonucleotides to the magnetic beads. Such surface coating may comprise reactive groups for conjugating to the polynucleotides/oligonucleotides and such reactive groups may be selected from the group consisting of carbodiimide (e.g. 1-Ethyl-3-(3-dimethylaminopropyl)carbodiimide (EDC)), amines (e.g., alkylamines), succinimides (such as N-hydroxy succinimide esters), imidates (e.g., imidoesters), imides (e.g. maleimide), haloacetyls, disulfides (e.g., pyridyldisulfide), hydrazines, diazirines or azides (such as aryl azides), avidins (e.g., streptavidin and Neutravidin), biotins, carboxyls, alkynes and thiols.

It is also to be understood that polynucleotides and/or oligonucleotides of the method of the invention may comprise a compound for conjugating to the surface coating of the magnetic bead. Such compounds may comprise reactive groups for conjugating to the polynucleotides/oligonucleotides and such reactive groups may be selected from the group consisting of carbodiimide (e.g. 1-Ethyl-3-(3-dimethylaminopropyl)carbodiimide (EDC)), amines (e.g., alkylamines), succinimides (such as N-hydroxysuccinimide esters), imidates (e.g., imidoesters), imides (e.g. maleimide), haloacetyls, disulfides (e.g., pyridyldisulfide), hydrazines, diazirines or azides (such as aryl azides), avidins (e.g., streptavidin and Neutravidin), biotins, carboxyls, alkynes and thiols.

The polynucleotide/oligonucleotide may be conjugated to the surface coating of the magnetic bead through click chemistry. For example, the surface of the magnetic bead may comprise an azide group and the polynucleotide/oligonucleotide may comprise an alkyne group which conjugates through click chemistry. For the avoidance of doubt, the conjugating groups may be switched around, for instance the magnetic bead surface may comprise an alkyne group and the polynucleotide/oligonucleotide may comprise an azide group.

Furthermore, the surface of the magnetic beads may comprise a layer, such as a silver or gold layer, to enhance the conjugation of the surface coating reactive groups to the magnetic bead surface.

Step (i) of the method of the invention involves providing a sample solution comprising a plurality of polynucleotides and/or oligonucleotides of interest. It is to be understood that the method may comprise a step prior to step (i) which includes the generation of the plurality of polynucleotides and/or oligonucleotides of interest as mentioned hereinbefore by appropriate amplification methods according to those known in the arts.

Alternatively, or additionally, following step (ii) of attaching the polynucleotides/oligonucleotides to the magnetic beads, the method may comprise a step of amplifying the bead-bound polynucleotides/oligonucleotides. In this sense, the polynucleotides/oligonucleotides that are bound to the beads for amplification may be padlock probes that are used to generate RCPs on the bead.

The beforementioned "amplification methods" include Polymerase Chain Reaction (PCR), Strand Displacement Assay (SDA), Transcription Mediated Assay (TMA), and single molecule amplification methods, such as Hybridization Chain Reaction (HCR) and, in particular, Rolling Circle Amplification (RCA).

RCA is a well-known single molecule amplification method that allows for digital quantification without compartmentalization. After labelling RCA products (may be referred to as "RCP" hereinafter) with molecules of defined optical properties such as fluorophores, said amplified molecules can be detected as single dots that can be quantified individually. Circular oligonucleotide templates to perform RCA may be designed and produced by a number of highly target specific means, and these targets may be virtually any nucleotide sequence.

RCA uses highly processive polymerases on a circular DNA target to generate a long ssDNA (i.e. single-stranded DNA) concatemer in hundreds of nanometers- to micrometer-range (Banér, J.; Nilsson, M.; Mendel-Hartvig, M.; Landegren, U. Signal Amplification of Padlock Probes by Rolling Circle Replication. Nucleic Acids Res. 1998, 26 (22), 5073-5078). RCA is often combined with "padlock probes" (PLPs), sequence specific oligonucleotides binding in a circular manner to the target strand which can then be covalently linked by a ligation step. A PLP-based RCA assay offers extreme stringency with single base precision (Nilsson, M.; Malmgren, H.; Samiotaki, M.; Kwiatkowski, M.; Chowdhary, B. P.; Landegren, U. Padlock Probes: Circularizing Oligonucleotides for Localized DNA Detection. Science. 1994, 265 (5181), 2085-2088). Similar to PLPs, "selector" probes may be combined with RCA, where the target is circularized prior to RCA (Johansson, H.; Isaksson, M.; Sörqvist, E. F.; Roos, F.; Stenberg, J.; Sjöblom, T.; Botling, J.; Micke, P.; Edlund, K.; Fredriksson, S.; Kultima, H. G.; Ericsson, O.; Nilsson, M. Targeted Resequencing of Candidate Genes Using Selector Probes. Nucleic Acids Res. 2011, 39 (2), e8).

As used herein, the term "rolling circle amplification products" refers to products generated by rolling circle amplification (RCA), such as long repetitive single-stranded amplicon consisting of hundreds of reverse complementary elements of a circular template, lined up in a single molecule. For the avoidance of doubt, polynucleotides and/or oligonucleotides generated by RCA may be referred to hereinafter as "RCA-products" or "RCP".

Hybridization Chain Reaction (HCR) is also a well-known single molecule amplification method that is similar to RCA, but does not rely on the use of enzymes for amplicon generation.

It is preferred that the polynucleotides and/or oligonucleotides in the method of the invention as defined hereinbefore are rolling circle amplification products or hybridization chain reaction products.

The inventors have found that the method typically arrives at only one polynucleotide or oligonucleotide being bound to one magnetic bead. Therefore, in an embodiment a single polynucleotide or oligonucleotide is bound to each magnetic bead. Without wishing to be bound by theory the inventors have two hypotheses for this occurrence. The first hypothesis is that the amplification of the polynucleotide or oligonucleotide occurs at a rate that it locally exhausts all reagents to start another amplification at the same location. The second hypothesis is that once the amplification product is formed it inhibits other amplification events from occurring by steric hindrance. In a similar manner, when the amplification products are already formed for capturing on the bead (rather than being formed on the bead) it is a stochastic process and due to the size of beads and amplification products, once on amplification product becomes bead bound it repels others from binding to the same bead.

In step (i) the sample solution comprises a plurality of polynucleotides and/or oligonucleotides that are not bead bound and, following step (ii) the plurality of polynucleotides and/or oligonucleotides are then bead-bound thus providing a further sample solution and, in this case, the sample solution in step (i) may also be referred to as a first sample solution and the sample solution prepared in step (ii) may be referred to as a second sample solution.

For the avoidance of doubt, in the method of the invention it is not necessary for all polynucleotides and/or oligonucleotides to become bead-bound in step (ii) to put the invention into practice and the skilled person will understand that due to thermodynamic and kinetic factors it is possible that not all polynucleotides and/or oligonucleotides will become bead-bound in the sample solution even if there is an excess of magnetic beads.

Step (iii) of the method of the invention involves applying the further sample solution containing the bead-bound polynucleotides/oligonucleotides to a first surface of a sample support element. That is to say, the sample support element comprises a first surface (e.g. a planar surface) onto which the further sample solution can be applied and retained in position on the sample support element. Such a support element may have a second surface opposite to the first surface.

The sample support element may comprise any material provided that it allows for the further sample solution to be applied and retained in place on a surface for further analysis/visualization. For example, the sample support element may be a microscope slide (e.g., a glass microscope slide) or a membrane. Alternatively, the first surface of the sample support element may form the bottom of a sample receiving well for receiving the further sample solution, optionally wherein the well comprises an aperture for introducing the further sample solution into the sample receiving well.

The amount of further sample solution added to the first surface of the sample support element may be in the range of from about 1 to about 50 µL, such as about 5 to about 20 µL.

Step (iv) of the method of the invention involves providing a magnetic source so as to draw (e.g. attract) the bead-bound polynucleotides to a position on the first surface of the sample support element.

According to the method of the invention, the magnetic source as defined hereinbefore (e.g. in step (iv) of the method of the invention) may draw (e.g. attract) the bead-bound polynucleotides/oligonucleotides to a position on the first surface of the sample support element that is equivalent to, or smaller than, the field of view of an optical sensing device. Alternatively, a funnel may be used to constrain the sample solution into multiple wells, wherein thereafter a magnetic source that spans multiple wells may be used to attract the bead-bound polynucleotides to multiple positions on the first surface of each well of the sample support element.

The magnetic source may be a permanent or non-permanent magnet, such as a neodymium magnet or an electromagnet. Furthermore, in the present invention, the magnetic source comprises an array of magnets or electromagnets, or a combination thereof, that are spatially configured around the sample support element so as to produce focused magnetic fields that draw (e.g. attract) the bead-bound polynucleotides/oligonucleotides to a position on the first surface of the sample support element.

The magnet may have a surface area that is facing the support element in the range of from about 0.75 mm² to about 25 cm², such as from about 0.75 mm² to about 12 cm², for example from about 7 mm² to about 12 cm².

The magnetic holding force (also commonly referred to as the pull force) of the magnetic source may be in the range of from about 1 g to about 50 kg, such as from about 1 g to about 500 g, for example from about 250 g to about 500 g. For the avoidance of doubt, the skilled person will understand that the magnetic holding force of a magnet is the force required to pull the magnet straight free from a 3.175 mm thick steel plate.

Following the provision of the magnetic source, the method may comprise an incubation step at room temperature to allow the bead-bound polynucleotides/oligonucleotides sufficient time to migrate towards the position on the first surface of the sample support element. For the avoidance of doubt, by the term "incubation" in this sense we mean that the sample is left undisturbed for a certain period of time and does not necessarily mean the sample is heated, for example the incubation may be at room temperature. However, the incubation may also be carried out under a controlled temperature, such as a temperature of from about 25 to about 50°C, such as about 25 to about 40°C.

It is to be noted that the step of providing a magnetic source includes the magnetic source already being present in the vicinity of the second surface of the sample support element when the further sample solution is applied. For example, the magnetic source may be a magnet that is fixed to the second surface of the sample support element meaning that when the sample solution is applied to the first surface the bead-bound polynucleotides/oligonucleotides immediately, or at least substantially immediately, begin being attracted towards the magnetic source.

Following step (iv) the bead-bound polynucleotides/oligonucleotides are visualized and/or quantified using a microscope, for example a fluorescence microscope, preferably an epifluorescence microscope. The method may, therefore, include a step of labelling the polynucleotides/oligonucleotides.

As explained in Examples 4 and 5, the inventors have unexpectedly found that the fluorescence signal of bead-bound polynucleotides/oligonucleotides is greater than the sum of the fluorescence of the beads and polynucleotides/oligonucleotides alone.

Various labels can be used including fluorophores, colorimetric labels, chemiluminescent labels, phosphorescent labels and particles, such as gold and silver particles, as well as quantum dots. For example the polynucleotides/oligonucleotides may be labelled with fluorescently tagged oligonucleotides or biotin tagged nucleotides. The polynucleotides/oligonucleotides may be labelled before, or after, binding to the beads.

Prior to visualization, the bead-bound polynucleotides/oligonucleotides may be washed. That is to say, after step (iv) of drawing (e.g. attracting) the bead-bound polynucleotides/oligonucleotides to a position on the first surface of the sample support element, a volume of the solution (i.e., the supernatant) may be removed and the bead-bound polynucleotides/oligonucleotides may be washed with a further solution.

The further solution for washing may comprise a surfactant, such as a polysorbate surfactant, for example polysorbate 20, which is also commonly referred to by the brand name Tween 20. The surfactant may be present in an amount of from about 0.1 to 5 % (v/v).

The solution for washing may also comprise salts, such as sodium chloride. The salt may be included in an amount of from about 20 to about 200 mM, such as from about 50 to about 150 mM.

The washing solution may also comprise a chelating agent, such as EDTA, optionally in an amount of from about 1 to about 20 mM, such as about 2 to about 10 mM.

The washing solution may further comprise a buffer, such as tris(hydroxymethyl)aminomethane (commonly referred to as Tris), optionally in an amount of from about 1 to about 20 mM, such as from about 5 to about 15 mM.

Alternatively, following removal of the volume of the solution, a further aliquot of the sample solution comprising bead-bound polynucleotides/oligonucleotides may be applied to the first surface of the sample support element after which the bead-bound polynucleotides/oligonucleotides in the further aliquot are also attracted to the position on the first surface of the sample support element. This allows for dilute samples to be concentrated in a quick and easy manner to allow visualization/quantification in a single field of view an optical sensing device. For the avoidance of doubt, following the addition of (a) further aliquot(s) of the sample solution, the bead-bound polynucleotides/oligonucleotides may be washed.

After being drawn (e.g. attracted) to the position on the first surface of the sample support element, the method may comprise the step of immobilising or fixing the bead-bound polynucleotides/oligonucleotides on the first surface of the sample support element. Once fixed the sample support element to be removed from the vicinity of the magnetic source for visualization. In this way, the magnet field does not have to be applied consistently to keep the bead-RCP complexes in position for subsequent imaging.

The bead-bound polynucleotides/oligonucleotides can be immobilized/fixed in a gel-like conformation after providing the magnetic source. Compounds that can be used to cast/create a gel may be selected from the group consisting of polyacrylamide, agarose, curing mounting media (e.g., VECTASHIELD Vibrance Antifade Mounting Media), UV curing chemicals, such as (meth)acrylate monomers, (meth)acrylated oligomers and photoinitiators (e.g., Diphenyl (2,4,6-trimethylbenzoyl) phosphine oxide (TPO)), and Epoxy, adhesives, e.g. cyanoacrylate based (Superglue) and silicone, or combinations of the above mentions gel chemistries.

It is also to be understood that polynucleotides and/or oligonucleotides or beads of the method of the invention may comprise a compound for conjugating to a coating on the first surface of the sample support element.

Such compounds for coating on the first surface of the sample support element may comprise reactive groups for conjugating to the polynucleotides/oligonucleotides or the beads, and such reactive groups may be selected from the group consisting of carbodiimide (e.g. 1-Ethyl-3-(3-dimethylaminopropyl)carbodiimide (EDC)), amines (e.g., alkylamines), succinimides (such as N-hydroxysuccinimide esters), imidates (e.g., imidoesters), imides (e.g. maleimide), haloacetyls, disulfides (e.g., pyridyldisulfide), hydrazines, diazirines or azides (such as aryl azides), avidins (e.g., streptavidin and Neutravidin), biotins, carboxyls, alkynes and thiols, or combinations thereof.

Also disclosed herein but not explicitly claimed is a sample analysis device as described hereinafter that enables polynucleotides and/or oligonucleotides of interest from a sample solution to be focused onto a small defined area that corresponds to the area of a single field of view of an optical sensing device, such as a microscope objective. The sample analysis device facilitates analysis of samples containing polynucleotides and/or oligonucleotides of interest with simple optical read-out, while still achieving a high detection sensitivity.

Therefore, also disclosed herein but not explicitly claimed is a sample analysis device comprising a sample support element having a first and second surface, wherein a magnetic source is attached to the second surface of sample analysis device.

The size of the magnetic source may be equivalent to, or smaller than, the field of view of an optical sensing device and may comprise any of the features as outlined above in respect of the first aspect of the invention.

The magnetic source may be an array of magnets or electromagnets, or a combination thereof, that are spatially configured around the sample support element so as to produce focused magnetic fields that draw (e.g., attract) the bead-bound polynucleotides/oligonucleotides to a position on the first surface of the sample support element.

The sample analysis device may be configured to enable polynucleotides and/or oligonucleotides of interest in a sample solution as defined herein to be focused into a small defined area that corresponds to the area of a single field of view of an optical sensing device, such as a microscope objective.

The sample analysis device may be used to enrich the polynucleotides and/or oligonucleotides of interest from a sample according to the method of the invention containing a low concentration of such polynucleotides and/or oligonucleotides onto the sensor detection zone so that detection of the polynucleotides and/or oligonucleotides requires only a single measurement that detects all polynucleotides and/or oligonucleotides of interest contained in the sample and thereby avoids the need to measure at several different areas on the field of detection and avoids using sophisticated imaging tools.

The sample analysis device is in particular designed to analyse and quantify polynucleotides/oligonucleotides generated (i.e. amplified) by rolling circle amplification.

The first surface of the sample support element according to the sample analysis device of the invention may form the bottom of a sample receiving well for receiving a sample molecule.

The sample receiving well according to the sample analysis device of the invention may comprise an aperture for introducing a sample solution into the sample receiving well. It is to be understood by the skilled person that such a well may also be an open well. Alternatively, the well may be covered, at least partially, preferably by an optically transparent material.

In the method of the invention the sample solution may be introduced into the well through the aperture and following step (iv) of attracting the bead-bound polynucleotides/oligonucleotides to a position on the first surface of the sample support element, the supernatant may be removed leaving the bead-bound polynucleotides/oligonucleotides in position followed by the introduction of a further aliquot of sample solution containing further bead-bound polynucleotides/oligonucleotides, or a washing solution (e.g., 10 mM Tris-HCl (pH 7.5), 5 mM EDTA, 100 mM NaCl and 0.1 % (v/v) Tween-20) may be introduced to wash the bead-bound polynucleotides/oligonucleotides.

Furthermore, the well may comprise an absorbent material positioned at one end of the well away from the area in which the bead-bound polynucleotides/oligonucleotides will be attracted to, and the absorbent material acts to draw in the sample solution through capillary forces thus allowing further sample solution to be added.

There is further disclosed herein, but not explicitly claimed, an alternative sample analysis device for use in the method of the invention, which sample analysis device comprises:
a sample support element comprising a plurality of wells for receiving a sample solution; and
a base element comprising a plurality of magnetic sources, wherein the base element is adapted so that the sample support element can be placed on top of the base element and wherein the plurality of magnetic sources are spatially configured to produce magnetic fields such that a focal point of the magnetic field is provided towards the centre of the bottom of each well in the sample support element.

For the avoidance of doubt, the bottom of each well in the sample support element is to be taken as the first surface of the sample support element as defined herein in relation to the method of the invention.

The sample support element and base element may be configured such that they are couplable in one orientation only. This ensures that the spatial arrangement of the magnetic sources is correct each time the sample support element is placed on top of the base element. For example, the sample support element and the base element may be shaped in a corresponding fashion such that they can only be coupled in one orientation. Alternatively, or additionally, the base element may comprise pins and the sample support element may comprise through holes, such that the sample support element will only fit on the base element if the pins and the through holes align.

This sample analysis device is useful in the present invention in that once sample solutions have been placed in each well, the sample support element can be placed on top of the base element and the bead-bound polynucleotides/oligonucleotides are drawn (e.g. attracted) to the centre of the bottom of each well. Following this, the sample support element can be removed and analysed. For example, the sample support element may be a 96 well plate that can be placed in a holder of a visualisation instrument allowing analysis of multiple samples.

As outlined above, the sample may be immobilized/fixed following the bead-bound polynucleotides/oligonucleotides being drawn (e.g. attracted) to the centre of the bottom of each well. To enable fixation, the bottom of each well in the sample support element may comprise a coating of reactive groups for conjugating to the polynucleotides/oligonucleotides or the beads. Suitable coating compounds are outlined above in respect of the method of the invention.

For the avoidance of doubt, although the two-part device is useful when it is desired to not have a magnet present during visualization, it is also contemplated that the sample within the wells can be visualized when the sample support element and the base element remain coupled.

In all embodiments when the sample analysis device is intended to be visualized in the presence of magnets, the bottom of the sample support element may comprise an opaque layer between the magnetic source to reduce fluorescence signal reflection and refraction from the magnet.

According to a second aspect of the invention, there is also provided a kit-of-parts comprising:
i) a container or plurality of containers comprising rolling circle amplification reagents and/or hybridization chain reaction reagents;
ii) a container comprising magnetic beads, such as the magnetic beads described herein; and
iii) a sample analysis device which comprises:
   a sample support element comprising a plurality of wells for receiving a sample solution; and
   a base element comprising a plurality of magnetic sources, wherein the base element is adapted so that the sample support element can be placed on top of the base element and wherein the plurality of magnetic sources are spatially configured to produce magnetic fields such that a focal point of the magnetic field is provided towards the centre of the bottom of each well in the sample support element;
   wherein the magnetic beads have an average size of from about 30 nm to about 200 nm.

According to a third aspect of the invention, there is also provided a kit-of-parts comprising:
i) a container or plurality of containers comprising rolling circle amplification reagents and/or hybridization chain reaction reagents;
ii) a container comprising magnetic beads, such as the magnetic beads described herein; and
iii) a sample analysis device comprising a sample support element having a first and second surface, wherein a magnetic source is attached to the second surface of the sample analysis device;
   wherein the magnetic beads have an average size of from about 30 nm to about 200 nm;
   wherein the magnetic source comprises an array of magnets or electromagnets, or a combination thereof, that are spatially configured around the sample support element so as to produce focused magnetic fields that draw (e.g., attract) the bead-bound polynucleotides/oligonucleotides to a position on the first surface of the sample support element.

Also disclosed herein but not explicitly claimed is a kit-of-parts comprising:
i) a container or plurality of containers comprising rolling circle amplification reagents and/or hybridization chain reaction reagents;
ii) a container comprising magnetic beads, such as the magnetic beads described herein; and
iii) instructions for use of the kit in the method according to the method of the first aspect of the invention.

The kit-of-parts according to the second or third aspect of the invention may further comprise a container comprising reagents for coating the first surface of the sample support element, which reagents comprise reactive groups for conjugating to the polynucleotides/oligonucleotides or the beads. Suitable compounds for coating the first surface of the sample support element are described above in respect of the method of the invention.

The kit-of-parts according to the second or third aspect of the invention may further comprise a container comprising reagents for casting/creating a gel for immobilizing the bead-bound polynucleotides/oligonucleotides. Suitable reagents for preparing such gels are outlined above in respect of the method of the invention.

Wherever the word 'about' is employed herein in the context of amounts, for example absolute amounts, such as weights, volumes, sizes, diameters, or relative amounts (e.g. percentages) of individual constituents in a composition or a component of a composition (including concentrations and ratios), timeframes, and parameters such as temperatures etc., it will be appreciated that such variables are approximate and as such may vary by ±10%, for example ±5% and preferably ±2% (e.g. ±1%) from the actual numbers specified herein. This is the case even if such numbers are presented as percentages in the first place (for example 'about 10%' may mean ±10% about the number 10, which is anything between 9% and 11%).

The embodiments, together with further objectives and advantages thereof, may best be understood by referring to the following description of the drawings taken together with the examples.

### Brief Description Of The Drawings

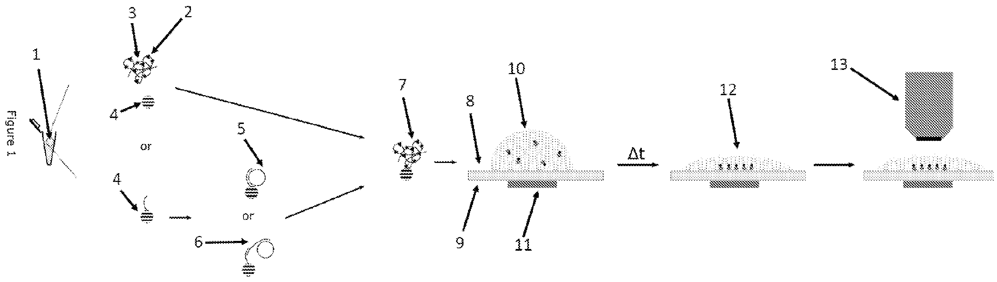

FIG. 1. Capture principle of an RCP, padlock probe or target DNA with padlock probe onto a carrier bead and side views of a cross section of an embodiment of the sample analysis module illustrating the main operations and principle of the sample analysis module. RCPs can either be captured after the RCA reaction, or the target DNA and/or the padlock probes can be captured on beads and amplified directly on them.

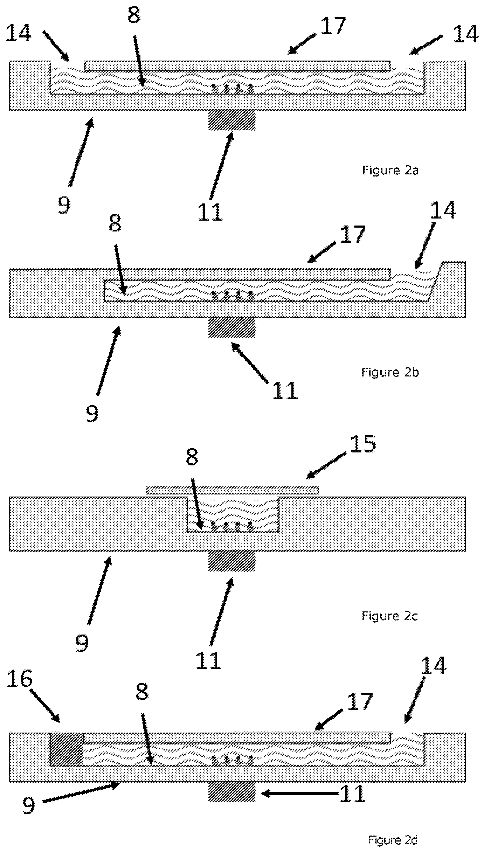

FIG. 2. Side views of cross sections of exemplary embodiments of the sample analysis module illustrating different design layouts. The use of open channels structures as well as the absorbent pad can increase the loading volume, thereby additionally increasing the detection sensitivity. A) Exemplary chip design 1 with 1 inlet and 1 outlet; B) Exemplary chip design 2 with 1 inlet and a chamber to hold the liquid; C) Exemplary chip design 3 with well, the well can be sealed with a cover slip prior image acquisition; and D) Exemplary chip design 4 with 1 inlet and an absorbent pad on the opposing end to allow more liquid to be filled inside the chip

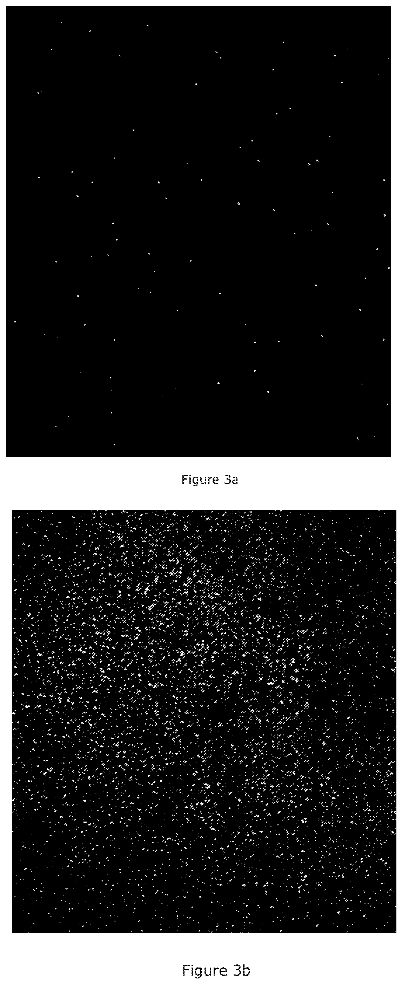

FIG. 3. Picture of fluorescently labeled RCPs immobilized (A) on a glass slide and (B) within the sample receiving well of an embodiment of the sample analysis module. A. showing 1 pM RCPs under a coverslip on a glass slide imaged with a 20x microscope objective. B. showing the same 1 pM RCP solution from (A) after use of the here described enrichment method with a 20x microscope objective.

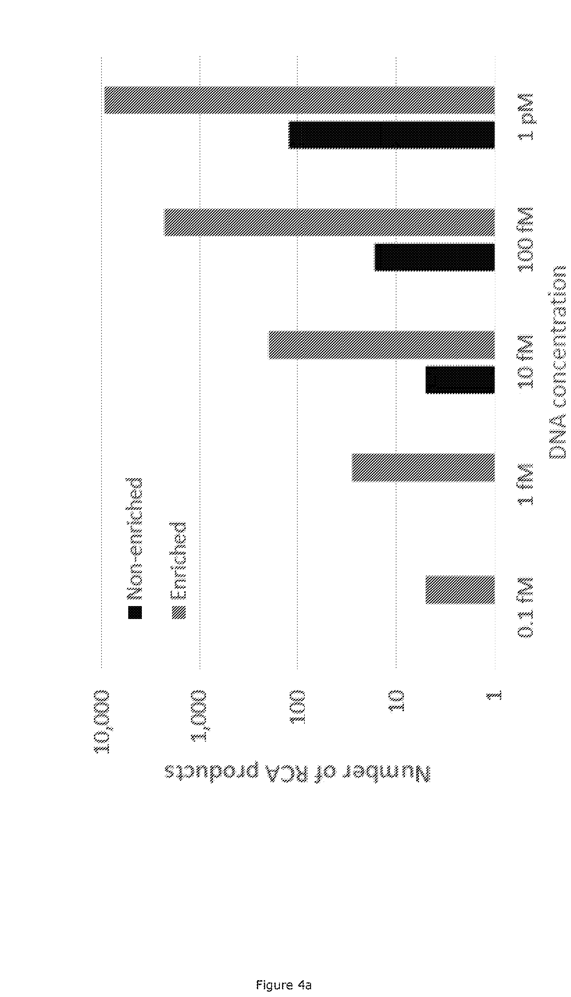

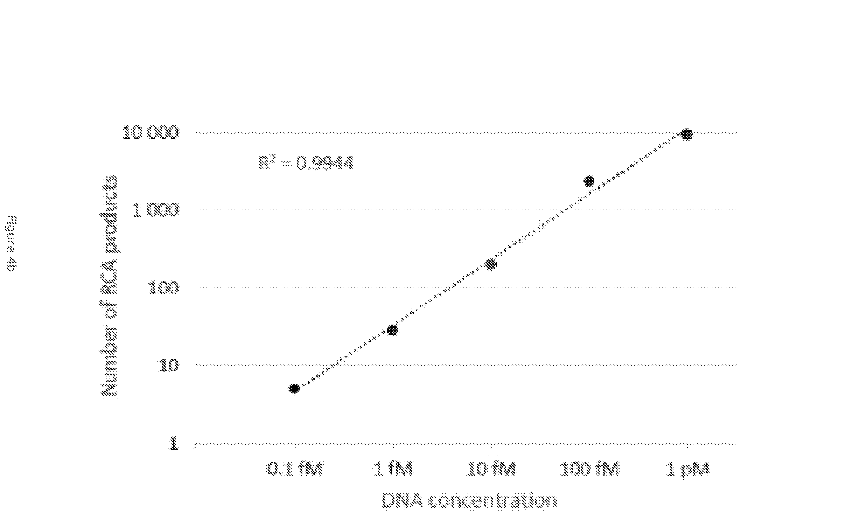

FIG. 4. Graphs illustrating quantification of serial dilutions of RCPs using an embodiment of the sample analysis module. A. showing the increased detection sensitivity compared to detection on a glass slide. B. showing the linear regression for the serial dilution. The average of two individual measurements is shown.

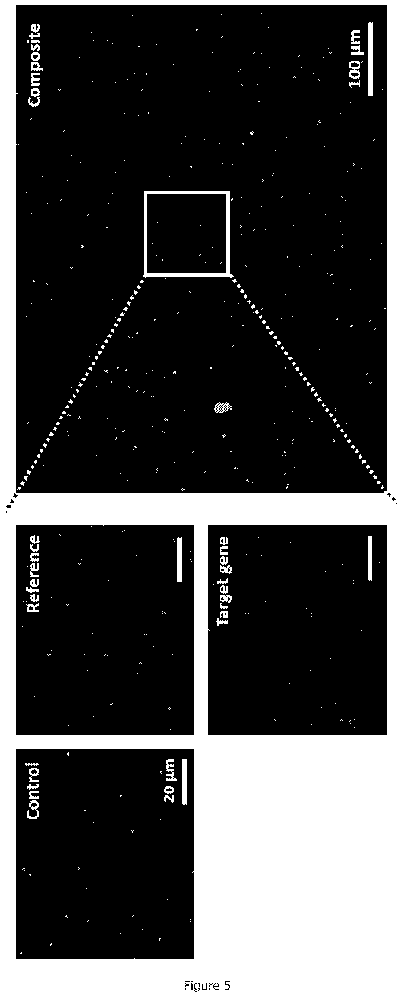

FIG. 5. Picture of fluorescently labeled RCPs from human genomic DNA immobilized by an embodiment of the sample analysis module. The RCPs are labelled with different fluorescent barcodes to distinguish the probes for the control, the target and the reference gene. The number of RCPs should be equal for all three genes (ratio 1:1:1), which is confirmed by the RCA in conjunction with the sample analysis module to visualize the low concentration of RCPs in the solution. Inset images show the respective RCPs for each of the fluorescent barcodes.

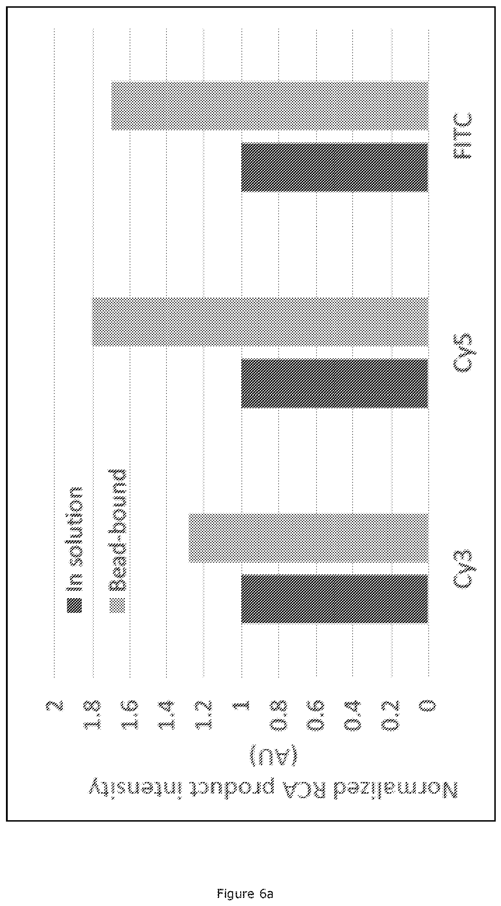

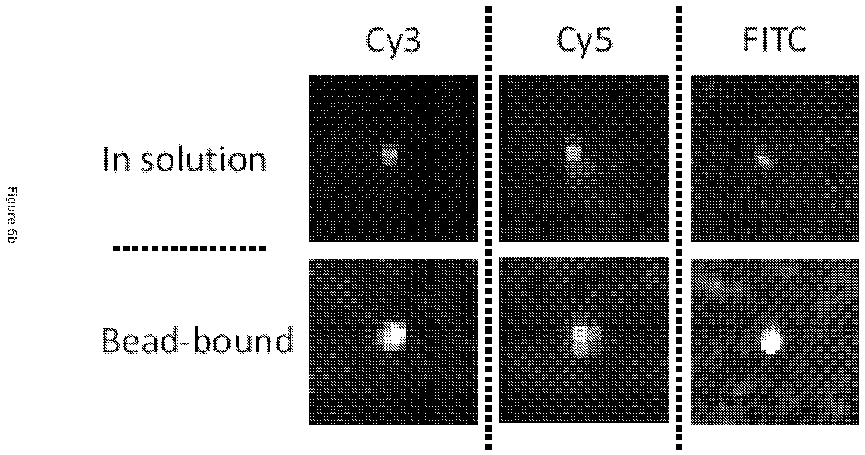

FIG. 6. Graph and images of exemplary RCPs illustrating the fluorescence intensity enhancing features of bead-bound RCPs compared to "free"/unbound RCPs. The enhancing properties are here exemplified by three different fluorescence channels. A. Graph of the fluorescence intensity of RCPs on slide and on beads. The population of RCPs stems from the same reaction, highlighting the increased intensity of bead-bound RCPs exemplary shown in three fluorescence channels. B. Image series showing single exemplary RCPs in solution (on a glass slide) and bead-bound. Images were set to the same thresholds to show the increased intensity and size of bead-bound RCPs.

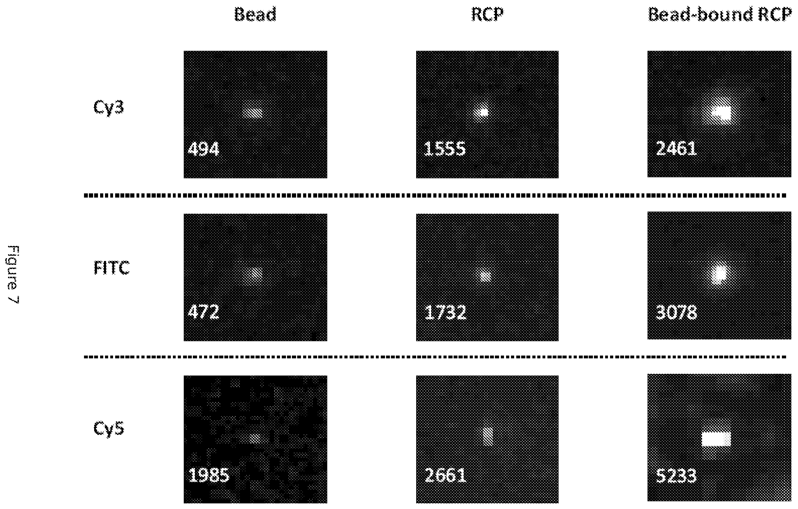

FIG. 7. Exemplary images showing that the fluorescence intensity of bead-bound RCPs is greater than the sum of beads and RCPs on their own. A. Exemplary images of magnetic beads, RCPs and bead-bound RCPs on a microscopy slide under a fluorescence microscope. The number in the left-hand corner corresponds to the highest fluorescence intensity of the image. B. Calculation of the exemplary images demonstrating the surprising fact that the sum of beads and RCPs is less than bead-bound RCPs.

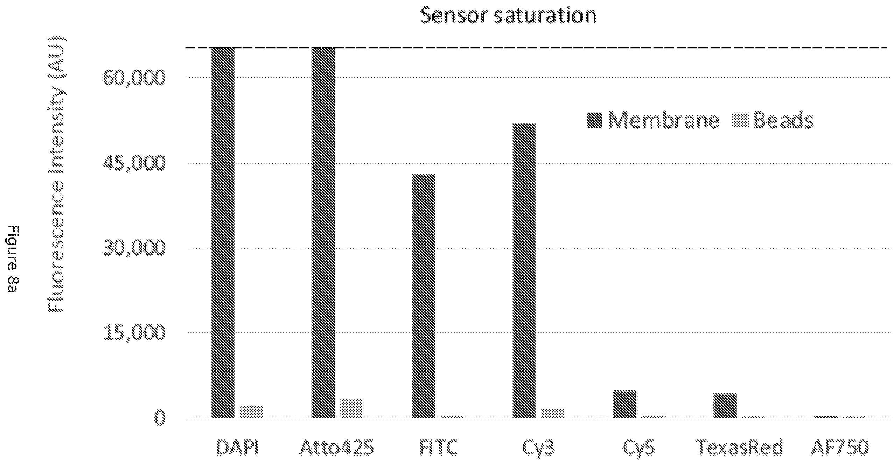

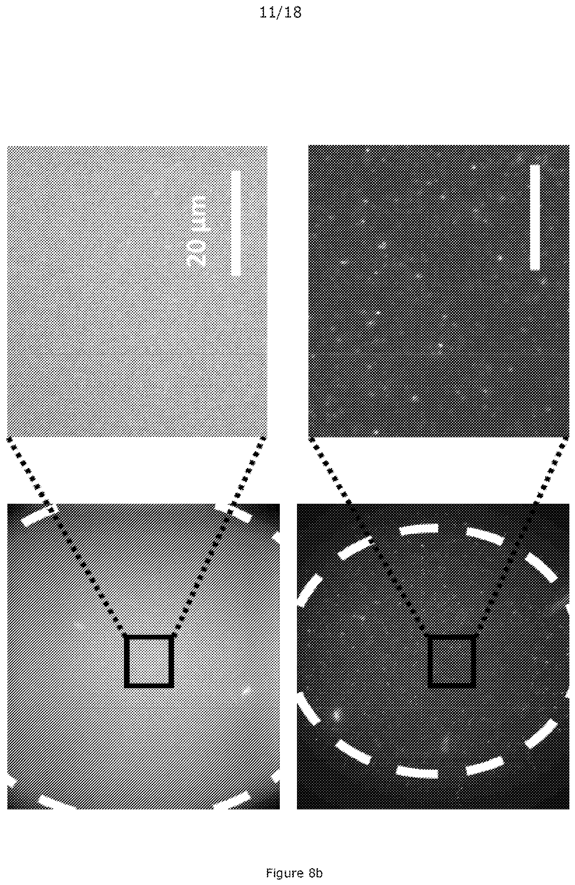

FIG. 8. Graph and images showing the autofluorescence of nitrocellulose membrane and magnetic beads in comparison. A. Graph of the autofluorescence levels of a nitrocellulose membrane and MyOne Dynabeads C1 for different fluorescence channels. B. Exemplary image and inset of FITC-labelled RCPs on membrane and bead bound. RCPs are clearly distinguishable with the magnetic enrichment using beads while the autofluorescence of nitrocellulose masks the RCPs.

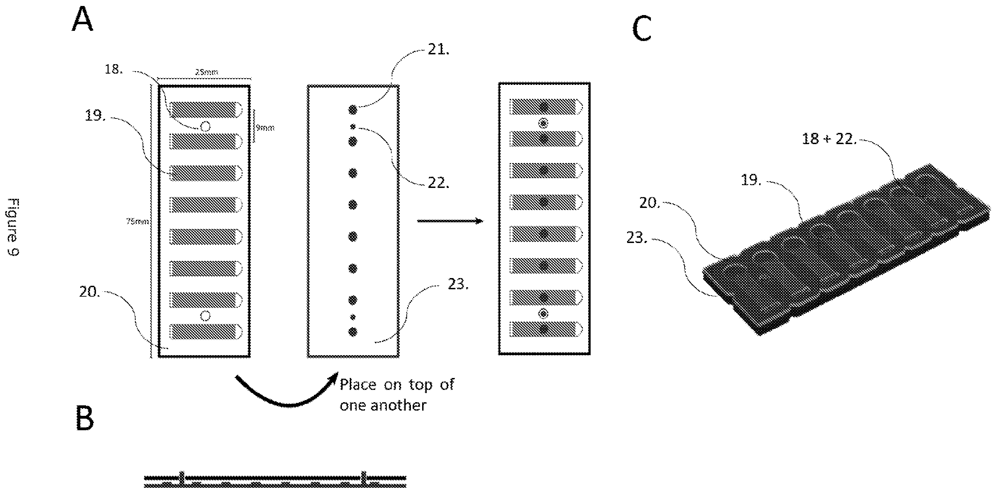

FIG. 9. Exemplary embodiment of a two-component sample analysis module with a (discardable) quantification chip and a reusable chip holder. A. Top view of an exemplary chip and chip holder design, and the assembly of both. B. Schematic side view of the exemplary assembled embodiment. C. Photograph of the assembled exemplary embodiment.

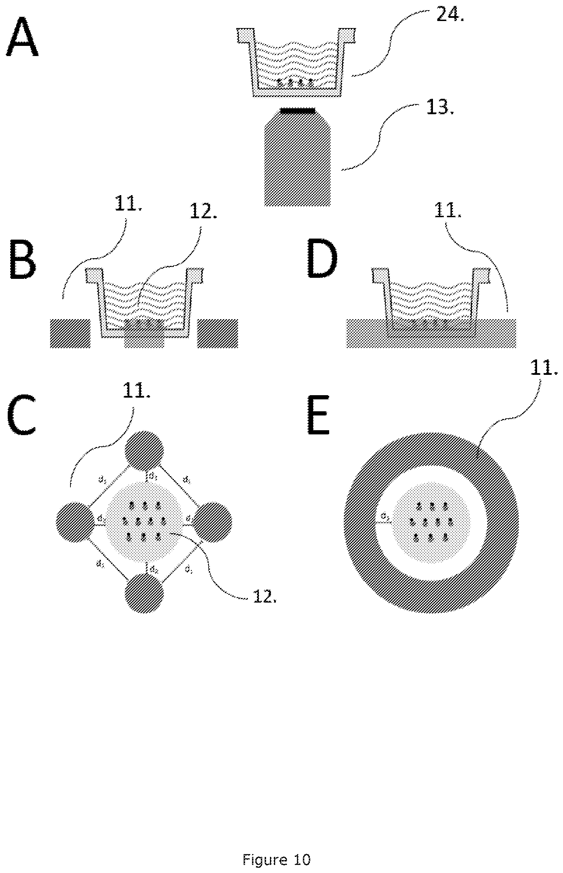

FIG. 10. Two exemplary concepts of magnet placement and design to create a homogeneous magnetic field in the center of the magnet or magnet arrangement. A. Exemplary schematic illustration of an inverted microscope for imaging through a well which would not work in the case of a magnet being placed between the chamber and the objective as it would block the view. B. and C. illustrate the arrangement of 4 magnets around the chamber D. and E. illustrate the arrangement of a single ring-shaped magnet to create a homogenous magnetic field in its center.

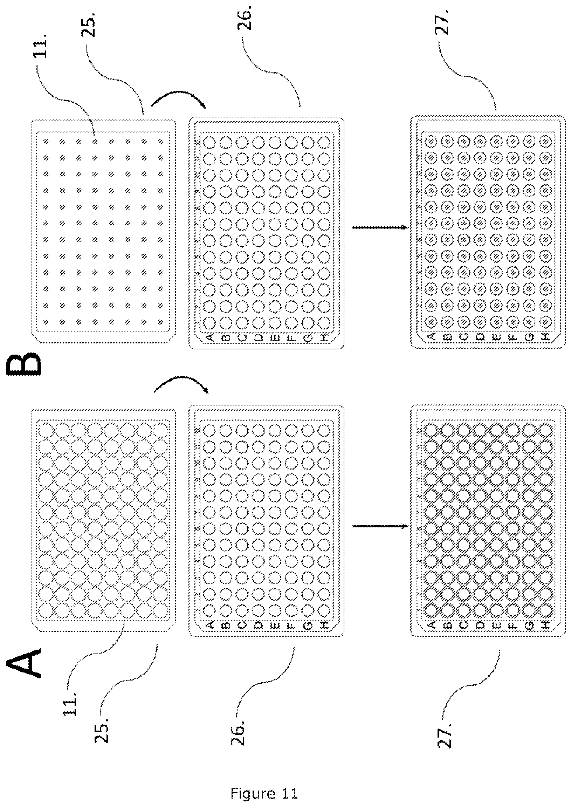

FIG. 11. Two exemplary multi-well designs to enable high throughput screening using the herein disclosed method. A. Illustration and example of a multi-well plate and plate holder housing ring-shaped magnetic sources as described in FIG. 3 D. and E. Fig. 11B. Illustration and example of a multi-well plate and plate holder housing disc-shaped magnetic sources.

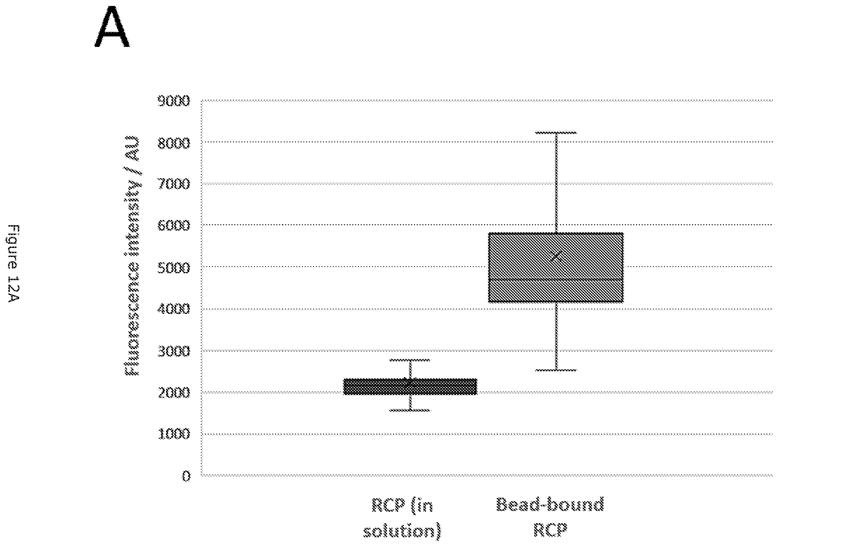

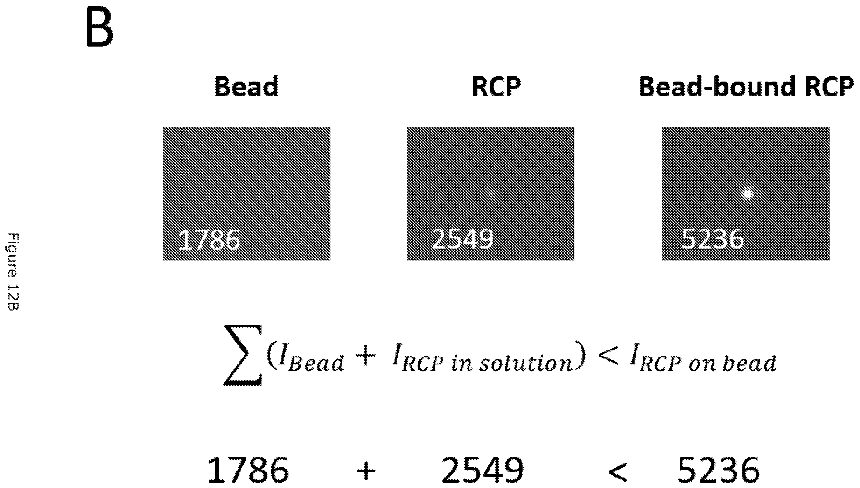

FIG. 12. Graph and images of exemplary RCPs illustrating the fluorescence intensity enhancing features of bead-bound RCPs compared to "free"/unbound RCPs and the independence on bead size (in a certain size range). A. Box plot of the fluorescence intensity of RCPs on slide and bound to beads. B. Image series showing single exemplary RCPs in solution (on a glass slide) and bead-bound (on the same glass slide).

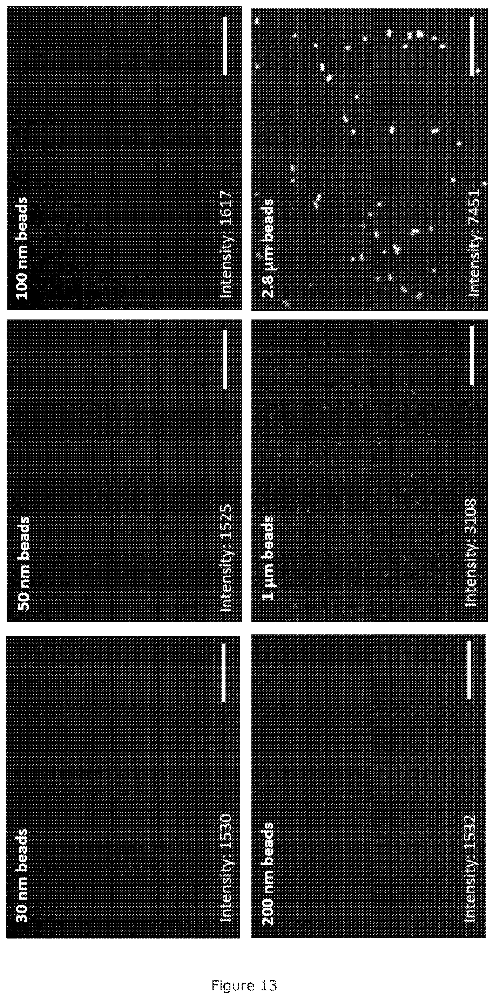

FIG. 13. Images of 6 exemplary bead sizes illustrating the non-trivial optical differences between them. Scale bar represents 40 µm.

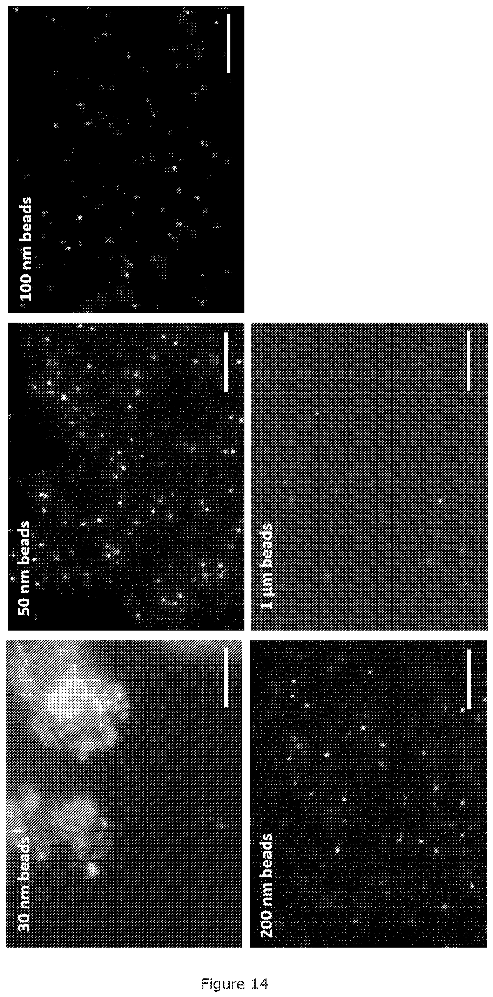

FIG. 14. Images of 5 exemplary bead sizes with RCPs bound to them and their non-trivial behavior and optical differences under magnetic force. Scale bar represents 20 µm.

In the figures, the following reference numbers have been used:
1. Solution containing Rolling Circle Amplification Product
2. Capture molecule (triangle shape in figure). Chemical or biological, e.g., thiol or biotin. Capture molecule can either be added by hybridization, reaction or during strand synthesis
3. Fluorescence dye (star shape in figure), e.g., sequence-specific or intercalating
4. Magnetic particle with functional groups. Chemical or biological (e.g., Streptavidin-functionalized or with capture oligonucleotide)
5. DNA circle or ligated padlock probe
6. Target DNA
7. Rolling Circle Amplification Product bound/immobilized on magnetic particle 7. Samples support element
8. First surface of the sample support element
9. Second surface of the sample support element
10. Solution with bead-bound Rolling Circle Amplification Products
11. Magnetic source, e.g., permanent or electrical
12. Rolling Circle Amplification products concentrated and magnetically immobilized on the surface
13. Microscope objective, e.g., 10x or 20x with a field of view matching the concentrated Rolling Circle Amplification Product area
14. Aperture
15. Thin glass, plastic layer or other transparent material to allow for short working distance imaging. Can be same material as the chip itself
16. Absorbent pad, e.g., paper or cotton
17. Transparent upper layer
18. Through hole
19. Enrichment chamber/channel
20. Enrichment chip
21. Magnet
22. Pin for holding chip in place
23. Chip holder
24. Well/chamber
25. Well/chamber plate holder housing the magnetic source
26. Well/chamber plate, e.g. 96-well plate
27. Well/chamber plate holder combined with Well/chamber plate

Throughout the drawings, the same reference numbers are used for similar or corresponding elements.

### Detailed Description of the Invention

The present invention is defined by the accompanying claims.

Figure 1 details the capture principle of an RCP, padlock probe or target DNA with padlock probe onto a carrier bead (4) and side views of a cross section of an embodiment of the sample analysis element illustrating the main operations and principle of the sample analysis module. RCPs can either be captured after the RCA reaction, or the target DNA and/or the padlock probes can be captured on beads and amplified directly on them.

The sample solution (1) comprising the rolling circle amplification products (7) is provided in an Eppendorf tube and magnetic particle (4) with functional groups (chemical or biological, e.g., Streptavidin-functionalized) or with capture oligonucleotides (2) are provided and added to the sample solution. Following a period of time to allow the rolling circle amplification products to bind to the magnetic beads, bead-bound RCPs were achieved (7) the sample solution was transferred to the first surface (8) of a glass slide acting as a sample support element.

A magnet (11) was then provided at a second surface (9) of the glass slide opposite to the first surface to attract the beads towards a certain area on the glass slide (12) that was equal to, or smaller than, the field of view of an optical imaging device (13), such as a fluorescence microscope.

Figure 2 shows four further embodiments of a sample analysis device according to the third aspect of the invention. Embodiment A shows the device wherein the first surface (8) of the support element forms the bottom of a well in which the sample solution is placed. The sample analysis device has an upper layer (17), which is transparent to allow visualization, and where the device comprises two apertures (14) to allow the sample solution to be added and removed as needed.

Embodiment B equates to embodiment A, but wherein the device comprises only one aperture (14). Embodiment C equates to embodiment A, but rather than having separate apertures, a glass cover slip (15) is provided over the top of the opening of the well to seal the volume.

Embodiment D is similar to embodiment A, but on one side of the device the aperture (14) is filled with an absorbent material (16) that absorbs excess sample solution through capillary forces to allow for further sample solution to be added once the bead-bound polynucleotides are held in place by the magnetic source.

Figure 9 shows an example of a two-component sample analysis module with a (discardable) quantification/enrichment chip (20) and a reusable chip holder (23). The quantification chip has through holes (18) that allow exact position and fit onto the chip holder pins (22). This allows imaging on up-right as well as inverted imaging systems. The 8 enrichment channels (19) are 9 mm apart from one another which allows loading with a standard multi-channel pipette. Figure 9A shows Top view of an exemplary chip (20) and chip holder (23) design, and the assembly of both. Figure 9B is a schematic side view of the exemplary assembled embodiment. Figure 9C is a photograph of the assembled exemplary embodiment. To reduce fluorescence signal reflection and refraction from the magnet (21), either the chip holder can be laminated with non-light absorbent paint or the enrichment chip itself has an opaque bottom layer; both cases have been explored with similar outcome.

Figure 10 shows two exemplary concepts of magnet (11) placement and design to create a homogeneous magnetic field in the center of the magnet or magnet arrangement. These magnet setups enable to create a homogeneous magnetic field for enrichment of the bead-bound RCPs while, at the same time, keeping the center of the chamber free to allow, e.g., image acquisition from the bottom (inverted microscopy). Figure 10A shows an exemplary schematic illustration of an inverted microscope (13) for imaging through a well (24) which would not work in the case of a magnet being place between the chamber and the objective as it would block the view. Figures 10B and 10C illustrate the arrangement of 4 magnets (11) around the chamber (side view and top view, respectively) to create a homogeneous magnetic field in the center of the magnet arrangement.

The arrangement and creation of such magnetic fields is well known and is described thoroughly in literature, such examples of which are: Tretiak, O., Blümler, P., & Bougas, L. (2019). Variable single-axis magnetic-field generator using permanent magnets. AIP Advances, 9(11), 115312. doi:10.1063/1.5130896; Manz, B., Benecke, M., & Volke, F. (2008); and a simple, small and low-cost permanent magnet design to produce homogeneous magnetic fields. Journal of Magnetic Resonance, 192(1), 131-138. doi:10.1016/j.jmr.2008.02.011.

The importance of such an arrangement is to ensure an equal distance between the magnets among one another (d1) as well as an equal distance of them to the chamber (d2). Figures 10D and 10E illustrate the arrangement of a single ring-shaped magnet to create a homogenous magnetic field in its center. By positioning the chamber with a solution containing RCPs (12) in the center of the magnet (d3), the RCPs will be enriched in the center. The two configurations illustrated in Figures 10B and 10C, as well as 10D and 10E, would both allow for inverted microscopy through the bottom layer of the chamber.

Figure 11 shows two exemplary multi-well designs to enable high throughput screening using the herein disclosed method. While Figure 3 shows an exemplary chip design in the size of a standard microscope slide (2.5 cm by 7.5 cm), here the exemplary plate is a standard 96-well plate to be able to fit into various standardized image acquisition units, such as microscopes and plate readers. Figure A is an illustration and example of a multi-well plate and plate holder housing ring-shaped magnetic sources as described in FIG. 3 D. and E.. This concept allows for the processing and analysis of 96 samples at a time. Figure B is an illustration and example of a multi-well plate and plate holder housing disc-shaped magnetic sources.

### Sequences

SEQ ID NO.: 1
   Padlock probe 1 
PO4-
GGGCAGCTGTCTAATTTTTGAGTCGGAAGTACTACTCTCTGTGTATGCAGCTCCTCAGTAATAGT
GTCTTACGTATCCTCGGAGAAGGTT
SEQ ID NO.: 2
   Synthetic target 1 
AGACCTGTTACATCTGGGTGCTTTCCTATAATGCACGACAGAACAAAAATTAGACAGCTGCCCAA
CCTTCTCCGAGGATAC
SEQ ID NO.: 3
   Detection probe for padlock probe 1
   Cy3-AGTCGGAAGTACTACTCTCT
SEQ ID NO.: 4
   Capture oligo
   Biotin-TTTTTCCTCAGTAATAGTGTCTTAC
SEQ ID NO.: 5
   RPP30 padlock probe 
PO4-
TTGTTGAGTGTTGGCGTGTATGCAGCTCCTCAGTAATAGTGTCTTACATTTAGCATACATCGTCG
CGTGCATAACCAGGCCA
SEQ ID NO.: 6
   NRXN1 unedited padlock probe 
PO4-
CGGCGGCCGCCTGCAGTGTATGCAGCTCCTCAGTAATAGTGTCTTACGGGCCTTATTCCGGTGC
TATGCTGATTCTGACGCG
SEQ ID NO.: 7
   NRXN1 reference padlock probe 
PO4-
AATAAGGGTCCCGAGGTGTATGCAGCTCCTCAGTAATAGTGTCTTACAGAGAGTAGTACTTCCGA
CTACACCGTGACGAAGA
SEQ ID NO.: 8
   Detection probe for RPP30
   Cy3-ATTTAGCATACATCGTCGCG
SEQ ID NO.: 9
   Detection probe for NRXN1 unedited
   FITC-GGGCCTTATTCCGGTGCTAT
SEQ ID NO.: 10
   Detection probe for NRXN1
   Cy5-AGAGAGTAGTACTTCCGACT
SEQ ID NO.: 11
   External primer
   TACTGAGGAGCTGCATAC*A*C
SEQ ID NO.: 12
   External primer
   ACACTATTACTGAGG
SEQ ID NO.: 13
   Detection probe 2 for NRXN1
   FITC-AGAGAGTAGTACTTCCGACT

Note that "*" denotes a phosphonothioate base.

### Examples

### List of Abbreviations

- RCA: = Rolling Circle Amplification
- DNA: = Deoxyribonucleic Acid
- BSA: = Bovine Serum Albumin
- dNTPs: = Deoxynucleotide triphosphates
- RCP(s): = Rolling Circle Amplification Product(s)
- EDTA: = ethylenediaminetetraacetic acid
- Tth: = Thermus Thermophilus
- NAD: = Nicotinamide Adenine Dinucleotide
- PBS: = Phosphate buffered saline

### Example 1

This example demonstrates the increased RCA product count per field of view using the invention when compared to a standard quantification on slide by spreading the RCA products under a cover slip. The example is shown in Figure 3 which shows that without being captured on magnetic beads and magnetically attracted to a predetermined position the number of RCA amplicons in a single field of view is much lower than those captured on magnetic beads.

### RCP production

Circular templates to serve for the subsequent RCA were generated by performing a padlock probe ligation reaction templated by a synthetic single-stranded DNA target mimicking that of a conserved 40 nt region of the *Hemagglutinin* gene from *Influenza B.* The ligation of padlock probes was performed with a mix composed of 100 pM padlock probes (PO4-GGGCAGCTGTCTAATTTTTGAGTCGGAAGTACTACTCTCTGTGTATGCAGCTCCTCAGTAATAGT GTCTTACGTATCCTCGGAGAAGGTT, SEQ ID NO: 1), 1 pM synthetic target (AGACCTGTTACATCTGGGTGCTTTCCTATAATGCACGACAGAACAAAAATTAGACAGCTGCCCA ACCTTCTCCGAGGATAC, SEQ ID NO: 2), Tth ligase buffer (20 mM Tris-HCl (pH 8.3), 25 mM KCl, 10 mM MgCl₂, 0.5 mM NAD, and 0.01% (v/v) Triton^{®} X-100) and 5 U Tth DNA ligase (Blirt S.A.) in a final volume of 20 µL. The mixture was incubated at 55 °C for 20 min.

Next, the resulting circles were amplified by target-primed RCA, for which a mixture comprising 0.2 µg/µL BSA (Fisher Scientific), 125 µM dNTPs (Fisher Scientific) and 8 U phi29 DNA polymerase (Blirt S.A.) in a final volume of 30 µL. The RCA reaction was incubated at 37 °C for 2 h and 65 °C for 2 min.

### Labelling of RCPs

The resulting RCPs were labelled using fluorescently tagged oligonucleotides and biotin tagged oligonucleotides complementary to the repeats within the RCPs. For this, the RCP products were mixed with 30 µL of labelling buffer (10 mM Tris-HCl (pH 8.0), 10 mM ethylenediaminetetraacetic acid (EDTA), 0.05% (v/v) Tween 20, 1 M NaCl containing 5 nM Cyanine 3 (Cy3)- (Cy3-AGTCGGAAGTACTACTCTCT, SEQ ID NO: 3) and biotin-tagged oligonucleotide (biotin-TTTTTCCTCAGTAATAGTGTCTTAC, SEQ ID NO: 4). The labelling reaction was incubated at 75 °C for 2 min and 55 °C for 15 min.

### Capturing RCPs on beads

The resulting labelled RCPs were captured on Dynabeads^{™}MyOne^{™} Streptavidin T1 (Thermo Fisher Scientific). Dynabeads^{™}MyOne^{™} Streptavidin T1 beads are superparamagnetic beads having a diameter of 1 µm, with a monolayer, not a multilayer, of recombinant streptavidin covalently coupled to the surface and further blocked with BSA. For this, the beads were prepared according to the manufacturer's instructions and subsequently added to the RCP solution at a concentration of 0.125 µg/µL. The capture reaction was incubated at 37 °C for 20 min and the bead subsequently washed once in washing buffer and resuspended in the same.

### Imaging of RCPs

To visualize the resulting bead-bound RCPs, 10 µL of the capture reaction were put on Superfrost glass slide (Thermo Fisher Scientific). A 1.5 mm circular magnet (Supermagnete) was attached to the slide to allow the local concentration of the bead-bound RCPs on a small surface area. To spread the solution, a 24 x 24 mm² coverslip was placed on top of the solution. The slide was incubated at room temperature for 5 min to allow the beads to be enriched. After incubation, the slide was imaged with an Olympus IX72 inverted fluorescence microscope with a 20x magnification objective and a field of view of 0.65 x 0.65 µm².

To visualize RCPs that were not captured on magnetic beads a similar procedure was used; however, no magnet was attached to the glass slide as the RCPs are not affected by magnetic force.

In conclusion, there is a striking difference in the number of RCPs observed in Figure 3a compared to Figure 3b. The number of RCPs is much higher in the same field of view when using the magnetic enrichment method as RCPs are attracted to a small surface area. The result is an increased sensitivity and, therefore, also simplified detection as samples containing low concentrations of RCPs do not need to be scanned. Thereby overcoming one of the major limitations of RCA which is the detection of RCPs at low concentrations.

### Example 2

### Analytical capabilities of the invention.

This example demonstrates the increased analytical capabilities using the invention when compared to a regular readout on slide. This example is illustrated in Figure 4.

RCPs were prepared the same way as described in the Example 1. In short, different synthetic target concentrations were circularized via ligation and amplified into RCPs for 2 h. Next, RCPs were labelled with a fluorescent and biotin probe.

For the enrichment method, the RCPs were incubated with magnetic beads as described in Example 1. The sample solution (10 µL of the 60 µL reaction volume) was applied to a cell counter slide (BioRad) which had a 1.5 mm magnet in diameter attached to its bottom. After 5 min, the cell counter slide was placed on the microscope stage and the enriched RCPs visualized using a 20x objective.

For the comparison method, the labelled RCPs were not captured on beads. The sample solution (10 µL of the 60 µL reaction volume) was applied to a cell counter slide, but no magnet attached. After 5 min the RCPs were settled down and could be visualized using the same 20x objective.

### RCP quantification

The resulting images were analyzed using a custom-made pipeline in the CellProfiler software (version 4.1.3; https://cellprofiler.org by the Broad Institute and initially published by Lamprecht et al. CellProfiler: free, versatile software for automated biological image analysis, Biotechniques (2007); 42(1):71-75). The pipeline consisted of image enhancement and object identification with manual thresholding.

In conclusion, the results confirm the increased sensitivity of the enrichment method disclosed herein when compared to a regular readout on a microscope glass slide. With the enrichment method, RCPs can be detected at concentrations where the regular readout appears blank (Figure 4a). Additionally, the number of detected RCPs correlates linearly with the concentration of input target. Thereby, confirming a concentration independent RCP enrichment (Figure 4b). Another benefit is that the regular pipeline for identifying RCPs can be used which makes the adaption of this method almost barrier-free.

### Example 3

### Quantification of human genomic DNA

This example demonstrates the capabilities to quantify different genes in genomic DNA and is illustrated in Figure 5. In this example, three different padlock probes were used to detect three different gene segments. One padlock probe served as the assay control if all steps were performed correctly (marked as Control); one padlock probe was used as reference (marked as Reference) to quantify the editing level when related to the gene of interest for the gene of interest; and, one padlock probe served as to identify the location of the gene edit (marked as Target gene). This means, in a wildtype experiment one would expect equal number of RCPs for all three padlock probes, while for an edited genome, one would expect equal number of RCPs for the Control and Reference but a reduced count for the Target gene. In this sample, we used wild type human genomic DNA, therefore, the number of RCPs for each of the padlock probes is equal.

This description is very generic as some cell lines might have varying chromosome or/and gene copy numbers. Therefore, it is advised to standardize a genome editing experiment against a wild-type sample and avoid potential biases.

### RCP production

Human genomic DNA (Merck) was used to generate circular templates for the RCA reaction. Three different regions on the genomic DNA were targeted, one region of *RPP30* gene and two regions on the *NRXN1* gene. First, 1 µg of human genomic DNA was fragmented in fragmentation mix consisting of buffer (20 mM Tris-HCl (pH 8.3), 25 mM KCl, 10 mM MgCl₂, 0.5 mM NAD, and 0.01% (v/v) Triton^{®} X-100) and 15 U AluI (New England Biolabs) in a total volume of 20 µL. The reaction was incubated at 37 °C for 5 min.

For the ligation, 10 µL of ligation mix were added containing Tth ligase buffer (20 mM Tris-HCl (pH 8.3), 25 mM KCl, 10 mM MgCl₂, 0.5 mM NAD, and 0.01% (v/v) Triton^{®} X-100), 1 nM of padlock probes (PO4-TTGTTGAGTGTTGGCGTGTATGCAGCTCCTCAGTAATAGTGTCTTACATTTAGCATACATCGTCG CGTGCATAACCAGGCCA, SEQ ID NO: 5; PO4-CGGCGGCCGCCTGCAGTGTATGCAGCTCCTCAGTAATAGTGTCTTACGGGCCTTATTCCGGTGC TATGCTGATTCTGACGCG, SEQ ID NO: 6; and, PO4-AATAAGGGTCCCGAGGTGTATGCAGCTCCTCAGTAATAGTGTCTTACAGAGAGTAGTACTTCCGA CTACACCGTGACGAAGA, SEQ ID NO: 7) and 7.5 U of Tth DNA ligase. The ligation reaction was incubated at 98 °C for 3 min and 55 °C for 45 min.

Next, the resulting circles were amplified by RCA, for which a mixture comprising 0.2 µg/µL BSA, 125 µM dNTPs, 5 nM external primer (TACTGAGGAGCTGCATAC*A*C, SEQ ID NO: 11; the star denotes a phosphonothioate base to escape exonucleic activity of the polymerase), 10.5 U exoI (New England Biolabs) and 28 U phi29 DNA polymerase in a final volume of 35 µL. The RCA reaction was incubated at 37 °C for 3 h and 65 °C for 2 min.

### Labelling of RCPs

The resulting RCPs were labelled using fluorescently tagged oligonucleotides and biotin tagged oligonucleotides as described in Example 1. In short, the RCP products were mixed with 15 µL of labelling buffer (10 mM Tris-HCl (pH 8.0), 10 mM ethylenediaminetetraacetic acid (EDTA), 0.05% (v/v) Tween 20, 1 M NaCl) containing 5 nM Cyanine 3 (Cy3)- (Cy3-ATTTAGCATACATCGTCGCG, SEQ ID NO: 8), biotin- (biotin-TTTTTCCTCAGTAATAGTGTCTTAC, SEQ ID NO: 4), AlexaFluor488 (FITC)- (FITC-GGGCCTTATTCCGGTGCTAT, SEQ ID NO: 9) and Cyanine 5 (Cy5)-tagged oligonucleotide (Cy5-AGAGAGTAGTACTTCCGACT, SEQ ID NO: 10). The labelling reaction was incubated at 75 °C for 2 min and 55 °C for 15 min.

### Capturing RCPs on beads & Imaging of RCPs

Capturing of the labelled RCPs, imaging and subsequent image analysis was done as described in Example 1 and 2.

In conclusion, Figure 5 shows a composite of all three channels. The insets show the RCPs separately for each of the three channels. As apparent, the number of RCPs for each channel is equal, thereby confirming the concept as this human genomic DNA should not carry any edit.

### Example 4

This example demonstrates the increased fluorescence intensity of bead-bound RCPs when compared to unbound/in-solution RCPs. The finding of this example is illustrated in Figure 6.

RCPs were generated and quantified as described in Example 3. For the analysis of the RCP fluorescence intensity, the CellProfiler pipeline was adapted to contain another module which measures the fluorescence intensity of each object.

The findings of this example illustrate that RCPs that are bound to a bead are brighter than unbound (Figure 6A). Furthermore, the increased fluorescence intensity made RCPs appear bigger when compared to RCPs in solution (Figure 6B). The comparison was based on the average of several hundred RCPs that were acquired in solution and bound to beads.

The conclusion drawn from this example is that the invention of binding and visualizing RCPs on magnetic beads results in an unexpected increased fluorescence intensity making the quantification easier, e.g. shorter exposure time needed and less sensitive optical device required.

### Example 5

This example confirms that bead-bound RCPs display a higher fluorescence intensity than the sum of blank magnetic beads (without a bound RCP) and un-bound RCPs. RCPs were generated and quantified as described in Example 3. For the exemplified calculation, blank magnetic beads (without a bound RCP), RCPs in solution and bead-bound RCPs were selected by hand and the maximum pixel intensity taken via ImageJ software. The sum of blank bead and in-solution RCP is smaller than the fluorescence intensity observed on bead-bound RCPs. This effect is exemplified with three fluorescence channels (Cy3, FITC and Cy5).

The fluorescence intensity results can be seen in Figure 7. When using Cy3 as the fluorescence label, the intensity of the signal from the bead alone was 494 and the RCP alone was 1555, whereas the bead-bound-RCP intensity was 2461 equaling an intensity increase of 20.1% compared to the sum of the bead and RCP alone.

When using FITC as the fluorescence label, the intensity of the signal from the bead alone was 472 and the RCP alone was 1732, whereas the bead-bound-RCP intensity was 3078 equaling an intensity increase of 39.7% compared to the sum of the bead and RCP alone.

When using Cy5 as the fluorescence label, the intensity of the signal from the bead alone was 1985 and the RCP alone was 2661, whereas the bead-bound-RCP intensity was 5233 equaling an intensity increase of 12.6% compared to the sum of the bead and RCP alone.

### Example 6

This example demonstrates the improved RCP quantification capability of the described method when compared to trapping of RCPs on a membrane. The example is illustrated in Figure 8.

The membrane and beads were imaged using a 20x objective in different fluorescence channels using the same exposure time. The membrane was wetted with PBS, and the beads eluted in PBS. The fluorescence intensity was measured using ImageJ software and taking the overall fluorescence intensity of the microscope image.

For the comparison of RCP intensities in Figure 8B, RCPs were generated as described in Example 3. Here, only the FITC-labelled RCPs are shown on the membrane and magnet as the difference is most apparent for shorter wavelengths. The top left image shows FITC-labelled RCPs on a nitrocellulose membrane and the zoom-in on the right illustrates that RCPs are not easily resolved due to the high autofluorescence. The image on the bottom left shows RCPs from the same solution but bound and enriched on beads. Apparent from the zoom-in on the bottom right, FITC-labelled RCPs can easily be resolved and quantified illustrating the advantages of the magnetic enrichment approach over the membrane one.

The membrane chip was manufactured by Aline, Inc. The filter membrane was a Protran^{™} NC Nitrocellulose membrane with a 0.1 pm pore size (GE Healthcare Life Sciences), the absorption layer was a cellulose fiber sample pad sheet (Merck), the spacing layer was in the form of pressure sensitive adhesive (Aline) and the liquid-impermeable layer was of polyethylene terephthalate (Aline). The sample receiving wells had a diameter of 1.5 mm. The sample analysis device was manufactured to have dimensions of a standard microscope slide 25x75 mm with ten sample receiving wells arrayed over the sample analysis device. The full features of this membrane chip are described in PCT/EP2020/060771 (published as WO 2020/212531).

For imaging and to concentrate the RCPs on the membrane, 10 µL of the sample solution were put on the sample receiving well. After the liquid passed through the membrane, 10 µL of Slowfade Gold (Fisher Scientific) were added on the membrane and a cover slip (Menzel) placed on top of it.

The benefits using the present inventive method to concentrate RCPs are:
- The bead-bound RCPs can be washed leading to lower background from assay or bio-sample components.
- The sensitivity seems to be increased per field of view when compared to spreading the solution on a glass slide, as bead-bound RCPs can be magnetically attracted to a small area matching that of the field of view of the imaging device.
- The spectral-labelling possibilities for this method are increased as the magnetic beads show low autofluorescence when compared to membrane enrichment.

### Example 7

This example confirms that bead-bound RCPs display a higher fluorescence intensity than the sum of blank magnetic beads (without a bound RCP) and un-bound RCPs.

Human genomic DNA (Roche) was used to generate circular templates for the RCA reaction. Three different regions on the genomic DNA were targeted, a region on the *GAPDH* gene, one region on the NRXN1 gene and on the *PLA3G6* gene. First, 1 µg of human genomic DNA was fragmented in fragmentation mix consisting of buffer (20 mM Tris-HCl (pH 8.3), 25 mM KCl, 10 mM MgCl₂, 0.5 mM NAD, and 0.01% (v/v) Triton^{®} X-100) and 2.5 U AluI (New England Biolabs) in a total volume of 20 µL. The reaction was incubated at 37 °C for 15 min.

For the ligation, 10 µL of ligation mix were added containing Tth ligase buffer (20 mM Tris-HCl (pH 8.3), 25 mM KCl, 10 mM MgCl₂, 0.5 mM NAD, and 0.01% (v/v) Triton^{®} X-100), 100 ng salmon sperm DNA (Thermo Fisher Scientific), 5 nM external primer (ACACTATTA CTGAGG, SEQ ID NO: 12), 1 nM of padlock probe (PO4-AATAAGGGTCCCGAGGTGTATGCAGCTCCTCAGTAATAGTGTCTTACAGAGAGTAGTACTTCCGA CTACACCGTGACGAAGA, SEQ ID NO: 7) and 1.26 U of Tth DNA ligase. The ligation reaction was incubated at 98 °C for 10 min and 55 °C for 20 min.

Next, the resulting circles were amplified by RCA, for which a mixture comprising 125 µM dNTPs, 4 U exoI (New England Biolabs) and 5.32 U phi29 DNA polymerase in a final volume of 35 µL. The RCA reaction was incubated at 37 °C for 2 h and 65 °C for 2 min.

### Labelling and capture of RCPs

The resulting RCPs were labelled using fluorescently tagged oligonucleotides and biotin tagged oligonucleotides complementary to the repeats within the RCPs. For this, the RCP products were mixed with 10 µL of labelling buffer (10 mM Tris-HCl (pH 8.0), 10 mM ethylenediaminetetraacetic acid (EDTA), 0.05% (v/v) Tween 20, 120 mM NaCl, 10 mM MgCl₂, containing 5 nM AlexaFluor488 (FITC)- (FITC-AGAGAGTAGTACTTCCGACT, SEQ ID NO: 13), biotin-tagged oligonucleotide (biotin-TTTTTCCTCAGTAATAGTGTCTTAC, SEQ ID NO: 4) and 0.025 µg/µL SuperMag Streptavidin magnetic beads, 50nm (Ocean NanoTech). The labelling reaction was incubated at 75 °C for 2 min and 55 °C for 10 min. The aforementioned SuperMag Streptavidin magnetic beads are superparamagnetic beads having a diameter of 50 nm, with a monolayer, not a multilayer, of recombinant streptavidin covalently coupled to the surface and further blocked with BSA.

### Capturing RCPs on beads & Imaging of RCPs

Capturing of the labelled RCPs, imaging was done as described in Example 1. The resulting images were analyzed using a custom-made pipeline in the ImageJ software. The pipeline consisted of image enhancement, object identification with manual thresholding, and fluorescence intensity measurement of each object.

This example confirms the surprising increase in fluorescence intensity of bead-bound RCPs in which the intensity exceeds the sum of blank magnetic beads and un-bound RCPs. For the exemplified calculation, blank magnetic beads, RCPs in solution and bead-bound RCPs were selected and the maximum pixel intensity taken via ImageJ software. The sum of blank bead and in-solution RCP is smaller than the fluorescence intensity observed on bead-bound RCPs.

The fluorescence intensity results can be seen in Figure 12. Figure 12A shows the increased fluorescence intensity for multiple RCPs, while Figure 12B illustrates this synergistic effect on an exemplary case. When using FITC as the fluorescence label, the exemplary intensity of the signal from the bead alone was 1786 and the RCP alone was 2549, whereas the bead-bound-RCP intensity was 5236 equaling an intensity increase of 20.8% compared to the sum of the bead and RCP alone.

### Example 8

This example confirms that 50 nm magnetic beads show the best performance in terms of low autofluorescence background, enrichment capability and binding efficiency.

For the comparison of fluorescence intensity of blank beads, 0.025 µg/µL of TurboBeads Streptavidin (Turbobeads GmbH), 0.025 µg/µL of aforementioned SuperMag Streptavidin magnetic beads of 50 - 200 nm (Ocean NanoTech), 0.125 µg/µL of the aforementioned Dynabeads^{™}MyOne^{™} Streptavidin T1 (Thermo Fisher Scientific), as well as 0.125 µg/µL Dynabeads^{™}M-270 Carboxylic Acid (Thermo Fisher Scientific), were prepared in Milli-Q water (Sigma-Aldrich). 10 µL of the sample were prepared for- and imaged on a microscope slide as described in Example 1, and quantified as described in Example 7. The exemplary images of bare nanoparticles are shown in Figure 13, while the nanoparticles with RCPs under a magnetic field (enriched) are shown in Figure 14.

When using the FITC channel, the 30 nm beads up to the 200 nm beads were not visible, while the 1 µm beads showed some level of autofluorescence and the 2.8 µm beads a high level of autofluorescence intensity. The intensity of the signal from the 30 - 200 nm in diameter beads were undistinguishable from the background, at around 1500 AU. For the Dynabeads^{™}MyOne^{™} Streptavidin T1, 1 µm in diameter, the fluorescence intensity was 3108 AU, whereas for the Dynabeads^{™}M-270 Carboxylic Acid, 2.8 µm in size, the fluorescence intensity was even higher at 7451 AU. These results are illustrated in Figure 13 with exemplary images.

To further evaluate the beads in terms of optical behavior and RCP capture and enrichment efficiency, in Figure 14 the beads were decorated with RCPs and enriched under a magnetic field, here the 30 nm Turbobeads showed high levels of autofluorescence and aggregation with intensity levels of around 6748 AU, which does not allow a quantification of RCPs. For 50 nm beads the number of RCPs in a single field of view is highest compared to 100 nm, 200 nm and 1 µm beads. Additionally, 1 µm beads start forming mosaic like structure when under magnetic force which increase the overall background intensity (noise) and makes image analysis of events more challenging. This shows that the ideal bead size for the shown beads is between 30 nm and 100 nm which is a counter intuitive result as often large particles (several µm to mm) are used to capture long polynucleotide sequences, e.g., in genomic extraction kits.

## Claims

1. A method of analysing a sample comprising a plurality of polynucleotides and/or oligonucleotides of interest, wherein the method comprises:
(i) providing a sample solution comprising a plurality of polynucleotides and/or oligonucleotides of interest;
(ii) attaching the polynucleotides/oligonucleotides to magnetic beads to provide bead-bound polynucleotides/oligonucleotides, thereby providing a further sample solution;
(iii) applying the further sample solution to a first surface of a sample support element; and
(iv) providing a magnetic source so as to draw (e.g. attract) the bead-bound polynucleotides/oligonucleotides to a position on the first surface of the sample support element;
wherein the magnetic source comprises an array of magnets or electromagnets, or a combination thereof, that are spatially configured around the sample support element so as to produce focused magnetic fields that draw (e.g., attract) the bead-bound polynucleotides/oligonucleotides to a position on the first surface of the sample support element;
wherein following step (iv) the bead-bound polynucleotides/oligonucleotides are visualized and/or quantified using a microscope; and
wherein the magnetic beads have an average size of from about 30 nm to about 200 nm.

2. The method according to Claim 1, wherein the magnetic beads are superparamagnetic beads, optionally comprising iron, nickel, cobalt or mixtures thereof.

3. The method according to any of Claims 1 or 2, wherein the plurality of polynucleotides/oligonucleotides are attached to the magnetic beads *via* adsorption or conjugation, or a combination thereof.

4. The method according to any preceding claim, wherein the magnetic beads comprise a surface coating configured for conjugating directly or indirectly (e.g., via a complementary capture oligonucleotide) to the polynucleotides/oligonucleotides.

5. The method according to Claim 4, wherein the surface coating comprises reactive groups for conjugating to the polynucleotides/oligonucleotides, optionally wherein the reactive groups are selected from the group consisting of carbodiimide (e.g. 1-Ethyl-3-(3-dimethylaminopropyl)carbodiimide (EDC)), amines (e.g., alkylamines), succinimides (such as N-hydroxysuccinimide esters), imidates (e.g., imidoesters), imides (e.g. maleimide), haloacetyls, disulfides (e.g., pyridyldisulfide), hydrazines, diazirines or azides (such as aryl azides), avidins (e.g., streptavidin and Neutravidin), biotins, carboxyls, thiols, alkynes and mixtures thereof.

6. The method according to Claim 4 or Claim 5, wherein the plurality of polynucleotides/oligonucleotides comprise a compound for conjugating to the surface coating of the magnetic beads.

7. The method according to Claim 6, wherein the compound for conjugating to the surface coating of the magnetic beads comprises reactive groups selected from the group consisting of carbodiimide (e.g. 1-Ethyl-3-(3-dimethylaminopropyl)carbodiimide (EDC)), amines (e.g., alkylamines), succinimides (such as N-hydroxysuccinimide esters), imidates (e.g., imidoesters), imides (e.g. maleimide), haloacetyls, disulfides (e.g., pyridyldisulfide), hydrazines, diazirines or azides (such as aryl azides), avidins (e.g., streptavidin and Neutravidin), biotins, carboxyls, alkynes, thiols, and mixtures thereof.

8. The method according to any preceding claim, wherein the magnetic source attracts the bead-bound polynucleotides/oligonucleotides to a position on the first surface of the sample support element that is equivalent to, or smaller than, the field of view of the microscope.

9. The method according to any preceding claim, wherein plurality of polynucleotides/oligonucleotides are rolling circle amplification products or padlock probes.

10. The method according to Claim 9, wherein the plurality of polynucleotides/oligonucleotides are prepared by a rolling circle amplification step or a hybridization chain reaction step.

11. A kit-of-parts comprising:
i) a container or plurality of containers comprising rolling circle amplification reagents and/or hybridization chain reaction reagents;
ii) a container comprising magnetic beads; and
iii) a sample analysis device which comprises:
a sample support element comprising a plurality of wells for receiving a sample solution; and
a base element comprising a plurality of magnetic sources, wherein the base element is adapted so that the sample support element can be placed on top of the base element and wherein the plurality of magnetic sources are spatially configured to produce magnetic fields such that a focal point of the magnetic field is provided towards the centre of the bottom of each well in the sample support element;
wherein the magnetic beads have an average size of from about 30 nm to about 200 nm.

12. A kit-of-parts comprising:
i) a container or plurality of containers comprising rolling circle amplification reagents and/or hybridization chain reaction reagents;
ii) a container comprising magnetic beads; and
iii) a sample analysis device comprising a sample support element having a first and second surface, wherein a magnetic source is attached to the second surface of the sample analysis device;
wherein the magnetic beads have an average size of from about 30 nm to about 200 nm;
wherein the magnetic source comprises an array of magnets or electromagnets, or a combination thereof, that are spatially configured around the sample support element so as to produce focused magnetic fields that draw (e.g., attract) the bead-bound polynucleotides/oligonucleotides to a position on the first surface of the sample support element.

13. The kit-of-parts according to Claim 12, wherein the size of the magnetic source is equivalent to, or smaller than, the field of view of an optical sensing device.

14. The kit-of-parts according to Claim 12 or Claim 13, wherein the first surface of the sample support element forms the bottom of a sample receiving well for receiving a sample solution.

15. The kit-of-parts according to Claim 14, wherein the sample receiving well comprises an aperture for introducing a sample solution into the sample receiving well.

## Patentansprüche

1. Verfahren zum Analysieren einer Probe, umfassend eine Vielzahl von Polynukleotiden und/oder Oligonukleotiden von Interesse, wobei das Verfahren umfasst:
(i) Bereitstellen einer Probenlösung, umfassend eine Vielzahl von Polynukleotiden und/oder Oligonukleotiden von Interesse;
(ii) Anbringen der Polynukleotide/Oligonukleotide an magnetischen Beads, um an Beads gebundene Polynukleotide/Oligonukleotide bereitzustellen, wodurch eine weitere Probenlösung bereitgestellt wird;
(iii) Aufbringen der weiteren Probenlösung auf eine erste Oberfläche eines Probenträgerelements; und
(iv) Bereitstellen einer Magnetquelle, um die an Beads gebundenen Polynukleotide/Oligonukleotide an eine Position auf der ersten Oberfläche des Probenträgerelements zu ziehen (z. B. anzuziehen);
wobei die Magnetquelle eine Anordnung von Magneten oder Elektromagneten oder eine Kombination davon umfasst, die räumlich um das Probenträgerelement herum konfiguriert sind, um fokussierte Magnetfelder zu erzeugen, welche die an Beads gebundenen Polynukleotide/Oligonukleotide an eine Position auf der ersten Oberfläche des Probenträgerelements ziehen (z. B. anziehen);
wobei nach Schritt (iv) die an Beads gebundenen Polynukleotide/Oligonukleotide unter Verwendung eines Mikroskops visualisiert und/oder quantifiziert werden; und
wobei die magnetischen Beads eine durchschnittliche Größe von etwa 30 nm bis etwa 200 nm aufweisen.

2. Verfahren nach Anspruch 1, wobei die magnetischen Beads superparamagnetische Beads sind, die optional Eisen, Nickel, Cobalt oder Mischungen davon umfassen.

3. Verfahren nach einem der Ansprüche 1 oder 2, wobei die Vielzahl von Polynukleotiden/Oligonukleotiden an die magnetischen Beads *über* Adsorption oder Konjugation oder eine Kombination davon gebunden ist.

4. Verfahren nach einem der vorstehenden Ansprüche, wobei die magnetischen Beads eine Oberflächenbeschichtung umfassen, die zum direkten oder indirekten Konjugieren (z. B. über ein komplementäres Capture-Oligonukleotid) an die Polynukleotide/Oligonukleotide konfiguriert ist.

5. Verfahren nach Anspruch 4, wobei die Oberflächenbeschichtung reaktive Gruppen zum Konjugieren an die Polynukleotide/Oligonukleotide umfasst, wobei optional die reaktiven Gruppen aus der Gruppe ausgewählt sind, bestehend aus Carbodiimid (z. B. 1-Ethyl-3-(3-dimethylaminopropyl)carbodiimid (EDC)), Aminen (z. B. Alkylaminen), Succinimiden (wie N-Hydroxysuccinimidestern), Imidaten (z. B. Imidoestern), Imiden (z. B. Maleimid), Haloacetylen, Disulfiden (z. B. Pyridyldisulfid), Hydrazinen, Diazirinen oder Aziden (wie Arylaziden), Avidinen (z. B. Streptavidin und Neutravidin), Biotinen, Carboxylen, Thiolen, Alkinen und Mischungen davon.

6. Verfahren nach Anspruch 4 oder 5, wobei die Vielzahl von Polynukleotiden/Oligonukleotiden eine Verbindung zur Konjugation an die Oberflächenbeschichtung der magnetischen Beads umfassen.

7. Verfahren nach Anspruch 6, wobei die Verbindung zum Konjugieren an die Oberflächenbeschichtung der magnetischen Beads reaktive Gruppen umfasst, ausgewählt aus der Gruppe, bestehend aus Carbodiimid (z. B. 1-Ethyl-3-(3-dimethylaminopropyl)carbodiimid (EDC)), Aminen (z. B. Alkylaminen), Succinimiden (wie N-Hydroxysuccinimidestern), Imidaten (z. B. Imidoestern), Imiden (z. B. Maleimid), Haloacetylen, Disulfiden (z. B. Pyridyldisulfid), Hydrazinen, Diazirinen oder Aziden (wie Arylaziden), Avidinen (z. B. Streptavidin und Neutravidin), Biotinen, Carboxylen, Alkinen, Thiolen und Mischungen davon.

8. Verfahren nach einem der vorstehenden Ansprüche, wobei die Magnetquelle die an Beads gebundenen Polynukleotide/Oligonukleotide an eine Position auf der ersten Oberfläche des Probenträgerelements anzieht, die dem Sichtfeld des Mikroskops entspricht oder kleiner als dieses ist.

9. Verfahren nach einem der vorstehenden Ansprüche, wobei Vielzahl von Polynukleotiden/Oligonukleotiden Rolling-Circle-Amplification-Produkte oder Padlock-Sonden sind.

10. Verfahren nach Anspruch 9, wobei die Vielzahl von Polynukleotiden/Oligonukleotiden durch einen Schritt der Rolling-Circle-Amplification oder einen Schritt der Hybridisierungskettenreaktion hergestellt werden.

11. Teilesatz, umfassend:
i) einen Behälter oder eine Vielzahl von Behältern, umfassend Rolling-Circle-Amplification-Reagenzien und/oder Hybridisierungskettenreaktionsreagenzien;
ii) einen Behälter, der magnetische Beads umfasst; und
iii) eine Probenanalysevorrichtung, die umfasst:
ein Probenträgerelement, umfassend eine Vielzahl von Vertiefungen zur Aufnahme einer Probenlösung; und
ein Basiselement, umfassend eine Vielzahl von Magnetquellen, wobei das Basiselement so angepasst ist, dass das Probenträgerelement oben auf dem Basiselement platziert werden kann, und wobei die Vielzahl von Magnetquellen räumlich konfiguriert ist, um Magnetfelder derart zu erzeugen, dass ein Brennpunkt des Magnetfelds in Richtung der Mitte des Bodens jeder Vertiefung in dem Probenträgerelement bereitgestellt wird;
wobei die magnetischen Beads eine durchschnittliche Größe von etwa 30 nm bis etwa 200 nm aufweisen.

12. Teilesatz, umfassend:
i) einen Behälter oder eine Vielzahl von Behältern, umfassend Rolling-Circle-Amplification-Reagenzien und/oder Hybridisierungskettenreaktionsreagenzien;
ii) einen Behälter, der magnetische Beads umfasst; und
iii) eine Probenanalysevorrichtung, umfassend ein Probenträgerelement mit einer ersten und einer zweiten Oberfläche, wobei eine Magnetquelle an der zweiten Oberfläche der Probenanalysevorrichtung angebracht ist;
wobei die magnetischen Beads eine durchschnittliche Größe von etwa 30 nm bis etwa 200 nm aufweisen;
wobei die Magnetquelle eine Anordnung von Magneten oder Elektromagneten oder eine Kombination davon umfasst, die räumlich um das Probenträgerelement herum konfiguriert sind, um fokussierte Magnetfelder zu erzeugen, die die an Beads gebundenen Polynukleotide/Oligonukleotide an eine Position auf der ersten Oberfläche des Probenträgerelements ziehen (z. B. anziehen).

13. Teilesatz nach Anspruch 12, wobei die Größe der Magnetquelle gleich dem oder kleiner als das Sichtfeld einer optischen Erfassungsvorrichtung ist.

14. Teilesatz nach Anspruch 12 oder 13, wobei die erste Oberfläche des Probenträgerelements den Boden einer Probenaufnahmevertiefung zur Aufnahme einer Probenlösung bildet.

15. Teilesatz nach Anspruch 14, wobei die Probenaufnahmevertiefung eine Öffnung zum Einführen einer Probenlösung in die Probenaufnahmevertiefung umfasst.

## Revendications

1. Procédé d'analyse d'un échantillon comprenant une pluralité de polynucléotides et/ou d'oligonucléotides d'intérêt, dans lequel le procédé comprend :
(i) la fourniture d'une solution d'échantillon comprenant une pluralité de polynucléotides et/ou d'oligonucléotides d'intérêt ;
(ii) la fixation des polynucléotides/oligonucléotides à des perles magnétiques pour fournir des polynucléotides/oligonucléotides liés à des perles, en fournissant de ce fait une autre solution d'échantillon ;
(iii) l'application de l'autre solution d'échantillon à une première surface d'un élément de support d'échantillon ; et
(iv) la fourniture d'une source magnétique de façon à entraîner (par exemple attirer) les polynucléotides/oligonucléotides liés à des perles à une position sur la première surface de l'élément de support d'échantillon ;
dans lequel la source magnétique comprend un réseau d'aimants ou d'électroaimants, ou une combinaison de ceux-ci, qui sont configurés spatialement autour de l'élément de support d'échantillon de façon à produire des champs magnétiques focalisés qui entraînent (par exemple, attirent) les polynucléotides/oligonucléotides liés à des perles à une position sur la première surface de l'élément de support d'échantillon ;
dans lequel à la suite de l'étape (iv) les polynucléotides/oligonucléotides liés à des perles sont visualisés et/ou quantifiés à l'aide d'un microscope ; et
dans lequel les perles magnétiques ont une taille moyenne allant d'environ 30 nm à environ 200 nm.

2. Procédé selon la revendication 1, dans lequel les perles magnétiques sont des perles superparamagnétiques, comprenant facultativement du fer, du nickel, du cobalt ou des mélanges de ceux-ci.

3. Procédé selon l'une quelconque des revendications 1 ou 2, dans lequel la pluralité de polynucléotides/oligonucléotides sont fixés aux perles magnétiques par l'intermédiaire d'une adsorption ou d'une conjugaison, ou d'une combinaison de celles-ci.

4. Procédé selon l'une quelconque revendication précédente, dans lequel les perles magnétiques comprennent un revêtement de surface conçu pour conjugaison directe ou indirecte (par exemple, par l'intermédiaire d'une capture d'oligonucléotide complémentaire) aux polynucléotides/oligonucléotides.

5. Procédé selon la revendication 4, dans lequel le revêtement de surface comprend des groupes réactifs pour conjugaison aux polynucléotides/oligonucléotides, facultativement dans lequel les groupes réactifs sont choisis dans le groupe constitué de carbodiimide (par exemple 1-éthyl-3-(3-diméthylaminopropyle)carbodiimide (EDC)), amines (par exemple, alkylamines), succinimides (tels qu'esters de N-hydroxysuccinimide), imidates (par exemple, imidoesters), imide (par exemple maléimide), haloacétyles, disulfures (par exemple, pyridyldisulfure), hydrazines, diazirines ou azotures (tels qu'azotures d'aryle), avidines (par exemple, streptavidine et Neutravidine), biotine, carboxyles, thiols, alcynes et mélanges de ceux-ci.

6. Procédé selon la revendication 4 ou la revendication 5, dans lequel la pluralité de polynucléotides/oligonucléotides comprennent un composé pour conjugaison au revêtement de surface des perles magnétiques.

7. Procédé selon la revendication 6, dans lequel le composé pour conjugaison au revêtement de surface des perles magnétiques comprend des groupes réactifs choisis dans le groupe constitué de carbodiimide (par exemple 1-éthyl-3-(3-diméthylaminopropyle)carbodiimide (EDC)), amines (par exemple, alkylamines), succinimides (tels qu'esters de N-hydroxysuccinimide), imidates (par exemple, imidoesters), imide (par exemple maléimide), haloacétyles, disulfures (par exemple, pyridyldisulfure), hydrazines, diazirines ou azotures (tels qu'azotures d'aryle), avidines (par exemple, streptavidine et Neutravidine), biotine, carboxyles, thiols, alcynes et mélanges de ceux-ci.

8. Procédé selon l'une quelconque revendication précédente, dans lequel la source magnétique attire les polynucléotides/oligonucléotides liés à des perles à une position sur la première surface de l'élément de support d'échantillon qui est équivalente au, ou plus petite que le, champ de vision du microscope.

9. Procédé selon l'une quelconque revendication précédente, dans lequel la pluralité de polynucléotides/oligonucléotides sont des produits d'amplification par cercle roulant ou des sondes cadenas.

10. Procédé selon la revendication 9, dans lequel la pluralité de polynucléotides/oligonucléotides sont préparés par une étape d'amplification par cercle roulant ou une étape de réaction en chaîne d'hybridation.

11. Trousse de pièces comprenant :
i) un récipient ou une pluralité de contenants comprenant des réactifs d'amplification par cercle roulant et/ou des réactifs de réaction en chaîne d'hybridation ;
ii) un récipient comprenant des perles magnétiques ; et
iii) un dispositif d'analyse d'échantillon qui comprend :
un élément de support d'échantillon comprenant une pluralité de puits permettant de recevoir une solution d'échantillon ; et
un élément de base comprenant une pluralité de sources magnétiques, dans laquelle l'élément de base est adapté de sorte que l'élément de support d'échantillon peut être placé au-dessus de l'élément de base et dans laquelle la pluralité de sources magnétiques sont configurées spatialement pour produire des champs magnétiques de telle sorte qu'un point focal du champ magnétique est fourni en direction du centre du fond de chaque puits dans l'élément de support d'échantillon ;
dans laquelle les perles magnétiques ont une taille moyenne allant d'environ 30 nm à environ 200 nm.

12. Trousse de pièces comprenant :
i) un récipient ou une pluralité de contenants comprenant des réactifs d'amplification par cercle roulant et/ou des réactifs de réaction en chaîne d'hybridation ;
ii) un récipient comprenant des perles magnétiques ; et
iii) un dispositif d'analyse d'échantillon comprenant un élément de support d'échantillon ayant une première et une seconde surface, dans laquelle une source magnétique est fixée à la seconde surface du dispositif d'analyse d'échantillon ;
dans laquelle les perles magnétiques ont une taille moyenne allant d'environ 30 nm à environ 200 nm ;
dans laquelle la source magnétique comprend un réseau d'aimants ou d'électroaimants, ou une combinaison de ceux-ci, qui sont configurés spatialement autour de l'élément de support d'échantillon de façon à produire des champs magnétiques focalisés qui entraînent (par exemple, attirent) les polynucléotides/oligonucléotides liés à des perles à une position sur la première surface de l'élément de support d'échantillon.

13. Trousse de pièces selon la revendication 12, dans laquelle la taille de la source magnétique est équivalente au, ou plus petite que le, champ de vision d'un dispositif de détection optique.

14. Trousse de pièces selon la revendication 12 ou la revendication 13, dans laquelle la première surface de l'élément de support d'échantillon forme le fond d'un puits de réception d'échantillon permettant de recevoir une solution d'échantillon.

15. Trousse de pièces selon la revendication 14, dans laquelle le puits de réception d'échantillon comprend une ouverture permettant d'introduire une solution d'échantillon dans le puits de réception d'échantillon.
